# EUROPEAN PATENT APPLICATION

(11) **EP 4 789 623 A1**
(43) Date of publication of application: **12.08.2026**
(21) Application number: 25156042.1
(22) Date of filing: 05.02.2025
(51) Int. Cl.: A61B 8/08, A61B 8/12, A61B 8/00

(54) **FETAL LIE AND FETAL SPINE POSITION USING ULTRASOUND IMAGING IN BLIND SWEEP PROTOCOL**

(71) Applicant: Koninklijke Philips N.V., 5656 AE Eindhoven (NL)
(72) Inventor: SARDAR, Mousumi, 5656 AG Eindhoven (NL); V, Manikanda Krishnan, 5656 AG Eindhoven (NL); VAJINEPALLI, Pallavi, 5656 AG Eindhoven (NL)
(74) Representative: Philips Intellectual Property & Standards

(57) **Abstract**

A system includes a processor configured for communication with an ultrasound probe. The processor is configured to control the ultrasound probe to obtain ultrasound image frames during a blind sweep protocol on a pregnant patient, and provide the ultrasound image frames as an input to a deep learning network trained to detect fetal anatomies. The processor is also configured to generate, as an output of the deep learning network, detections of the fetal anatomies of a fetus within the ultrasound image frames, determine a fetal lie using the detection of the fetal anatomies, and display an output representative of the fetal lie.

## Description

### FIELD OF THE INVENTION

The subject matter described herein relates to devices, systems, and methods for using ultrasound data from a blind abdominal imaging sweeps to characterize fetal position (e.g., fetal lie and/or fetal spine position).

### BACKGROUND OF THE INVENTION

Ultrasound imaging is often used for diagnostic purposes in an office or hospital setting, but may also be used in resource-constrained care settings (e.g., homes, accident sites, ambulances, mobile health facilities, etc.) by emergency personnel, home health nurses, midwives, etc., who may lack ultrasound expertise. To facilitate ultrasound image acquisition by untrained or minimally trained users, a "blind sweep" protocol is often employed, in which the user follows pre-determined probe paths (e.g., sweeping out a pattern on the patient's abdomen) during imaging.

Ultrasound imaging is a vital component of high-quality obstetric care. For example, pregnancy requires constant monitoring by health care providers to avoid conditions that may threaten the lives of the fetus and the mother at birth. For labor management, the diagnosis of fetal orientation (including fetal presentation and fetal lie) is essential to guarantee delivery viability. A direct indicator of fetal presentation is the fetal head location, which can be placed close to the canal birth (cephalic, head-first) or far from the canal birth (breech, feet first). Unlike urban areas, the population in rural zones experience difficulties in accessing healthcare monitoring. Although telemedicine has helped bring medical technology closer to these regions, the diagnosis still requires medical specialists.

The information included in this Background section of the specification, including any references cited herein and any description or discussion thereof, is included for technical reference purposes only and is not to be regarded as subject matter by which the scope of the disclosure is to be bound.

### SUMMARY OF THE INVENTION

The invention is defined by the claims. Advantageous embodiments are provided in the dependent claims. Disclosed is a fetal orientation detection system that, following a blind sweep protocol (sweeps at particular locations along body without trying to find specific anatomy), detects anatomical features in the captured images and uses them for an automatic workflow of fetus characterization (including fetal lie and presentation) and monitoring of the labor/fetus evolution, through an anatomical spatio-temporal map, constructed from a deep-learning anatomical detector/segmentation model using imaging data from a blind sweep. The workflow also includes sweep calibration steps which help user to ensure complete capture of fetal anatomies for correct assessment.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a system with a processor configured for communication with an ultrasound probe, where the processor is configured to: control the ultrasound probe to obtain a plurality of ultrasound image frames during a blind sweep protocol on a pregnant patient; provide the plurality of ultrasound image frames as an input to a deep learning network trained to detect a plurality of fetal anatomies; generate, as an output of the deep learning network, a plurality of detections of the plurality of fetal anatomies of a fetus within the plurality of ultrasound image frames; determine a fetal lie using the detection of the plurality of fetal anatomies; and provide, to a display in communication with the processor, an output representative of the fetal lie. Other embodiments of this aspect include corresponding computer systems, apparatus, and computer programs recorded on one or more computer storage devices, each configured to perform the actions of the methods. In particular, another general aspect includes method for characterizing a fetal position using ultrasound data from a blind abdominal imaging sweep, comprising: controlling an ultrasound probe to obtain a plurality of ultrasound image frames during a blind sweep protocol on a pregnant patient; providing the plurality of ultrasound image frames as an input to a deep learning network trained to detect a plurality of fetal anatomies; generating, as an output of the deep learning network, a plurality of detections of the plurality of fetal anatomies of a fetus within the plurality of ultrasound image frames; determining a fetal lie using the detection of the plurality of fetal anatomies; and providing, to a display, an output representative of the fetal lie. Another general aspect includes a non-transitory computer-readable medium storing instructions that, when executed by a processor, cause the processor to perform the above method.

Implementations may include one or more of the following features. In some aspects, the fetal lie may include one of a longitudinal lie, a transverse lie, or an oblique lie. In some aspects, the longitudinal lie may include cephalic or breech. In some aspects, the transverse lie may include: fetal head on maternal right; or fetal head on maternal left. In this manner, it is possible to advantageously increase coverage of a zone of interest for an obstetric scan. The system's ability to identify specific fetal lie types (such as longitudinal, transverse, or oblique) may allow for detailed diagnosis and customized care plans during pregnancy. On the other hand, pinpointing the longitudinal lie as either cephalic or breech may afford precise monitoring, helping in planning interventions or deliveries accordingly.

In some aspects, the blind sweep protocol may include a vertical sweep, where the processor is configured to: generate a plurality of first binary masks based on the plurality of detections in the vertical sweep, where an individual first binary mask is associated with an individual ultrasound image frame in the vertical sweep; and generate a first map based on the plurality of first binary masks, where the first map is representative of the vertical sweep as a whole, where the processor is configured to determine the fetal lie using the first map. By means of a vertical sweep, a comprehensive first map from binary masks can be generated, which advantageously may enhance accuracy in determining the fetal lie, and lead to more reliable assessments. In some aspects, the output representative of the fetal lie may include the first map. This provides a visual representation of the fetal lie, advantageously simplifying the understanding and communication of the fetus's position.

In some aspects, the processor is configured to: generate a report which may include the first map and a previous first map; and output to the report to the display, where the first map and the previous first map each may include a region representative of a fetal anatomy, where a size of the region in the first map relative to a size of the region in the previous first map is configured to provide indication of a growth of the fetus. Including the first and previous maps in a report may advantageously allow tracking fetal growth over time, offering crucial data for monitoring health and development. In some aspects, the first map may include a first region representative of a first fetal anatomy and a second region representative of a different, second fetal anatomy, where, to determine the fetal lie using the first map, the processor is configured: perform principle component analysis (PCA) on the first map; generate, based on the PCA, a line between the first region and the second region; and determine an orientation of the line; and determine the fetal lie based on the orientation of the line. In particular, the use of PCA to analyze the first map may ensure precise determination of the fetal anatomy orientation, advantageously aiding in accurate assessment of fetal lie. In some aspects, the blind sweep protocol may include a horizontal sweep, where, when the processor determines the fetal lie is a transverse lie, the processor is configured to determine, based on the horizontal sweep, if the transverse lie is: fetal head on maternal right; or fetal head on maternal left. By implementing a horizontal sweep, it is possible to identify the specific type of transverse lie, which is critical in deciding the best management for the pregnancy.

In some aspects, the map may include a first region representative of a fetal head, where, to determine if the transverse lie is fetal head on maternal right or fetal head on maternal left, the processor is configured to: generate a plurality of second binary masks based on the plurality of detections in the horizontal sweep; generate a second map based on the plurality of second binary masks, where the second map is representative of the horizontal sweep; and generate a horizontal mid-line in the second map; determine that the transverse lie is: fetal head on maternal right when the first region is below the horizontal mid-line; and fetal head on maternal left when the first region is above the horizontal mid-line. Such a detailed process of determining whether the fetal head is on the maternal right or left may advantageously provide an improved clarity and exactness in fetal positioning, which is helpful in planning deliveries. In some aspects, the deep learning network is trained to detect a plurality of maternal anatomies, where the processor is further configured to: generate, as the output of the deep learning network, a plurality of detections of the plurality of maternal anatomies within the plurality of ultrasound image frames; and determine a fetal spine position using the plurality of detections of the plurality of fetal anatomies and the plurality of detections of the plurality of maternal anatomies, and where the output is representative of the fetal spine position. Thus, at least in some of these aspects, the deep learning network is additionally trained to detect a plurality of maternal anatomies, a plurality of detections of the plurality of maternal anatomies within the plurality of ultrasound image frames is generated also as the output of the deep learning network, and the output is additionally representative of the fetal spine position. It has been found that training the deep learning network also to detect maternal anatomies advantageously improves the system's ability to accurately determine fetal spine position, further enhancing the overall diagnostic capability. In some aspects, the processor is configured to: control the ultrasound probe to perform a calibration sweep before the blind sweep protocol; determine if a length of the calibration sweep is acceptable; prompt a user to rescan when the length of the calibration sweep is not acceptable; and prompt the user to proceed to the blind sweep protocol when the length of the calibration sweep is acceptable. This may advantageously improve the reliability of the results because, by performing a calibration sweep, the accuracy of subsequent scans can be ensured, which supports better clinical decisions.

Implementations of the described techniques may include hardware, a method or process, or computer software on a computer-accessible medium.

One general aspect includes a system with a processor configured for communication with an ultrasound probe, where the processor is configured to: control the ultrasound probe to obtain a plurality of ultrasound image frames during a blind sweep protocol on a pregnant patient; provide the plurality of ultrasound image frames as an input to a deep learning network trained to detect a plurality of fetal anatomies and a plurality of maternal anatomies; generate, as an output of the deep learning network, a plurality of detections of the plurality of fetal anatomies and a plurality of detections of the plurality of maternal anatomies within the plurality of ultrasound image frames; determine a fetal spine position using the plurality of detections of the plurality of fetal anatomies and the plurality of detections of the plurality of maternal anatomies; and provide, to a display in communication with the processor, an output representative of the fetal spine position. Other embodiments of this aspect include corresponding computer systems, apparatus, and computer programs recorded on one or more computer storage devices, each configured to perform the actions of the methods.

Implementations may include one or more of the following features. In some aspects, the fetal spine position may include anterior or posterior. Knowing whether the fetal spine is in the anterior or posterior position may advantageously help healthcare providers to more accurately assess fetal orientation and plan for labor, potentially reducing complications. In some aspects, the processor is configured to: determine a plurality of mid-points for the plurality of detections of the plurality of fetal anatomies and the plurality of detections of the plurality of maternal anatomies; and generate a plot of the plurality of mid-points; where the processor is configured to determine the fetal spine position based on the plot of the plurality of mid-points. In some aspects, the plot may include: a first cluster of the plurality of mid-points representative of a first fetal anatomy; a second cluster of the plurality of mid-points representative of a second fetal anatomy; and a third cluster of the plurality of mid-points representative of a first maternal anatomy, where the processor is configured to determine, in the plot, a first centroid of the first cluster, a second centroid of the second cluster, and a third centroid of the third cluster, where the processor is configured to determine the fetal spine position based on a relative positioning of the first centroid, the second centroid, and the third centroid. In some aspects, the first fetal anatomy may include a fetal spine, where the second fetal anatomy may include a fetal heart, and where the first maternal anatomy may include a placenta, and where the relative positioning may include the second centroid associated with the fetal heart being positioned between the first centroid associated with the fetal spine and the third centroid associated with the placenta. In some aspects, the plot may include a fourth cluster of the plurality of mid-points representative of a second maternal anatomy, where the second maternal anatomy may include amniotic fluid, where the processor is configured to determine, in the plot, a fourth centroid of the fourth cluster, where the processor is configured to determine the fetal spine position based on a placenta location identified using a relative positioning of the third centroid and the fourth centroid. In some aspects, the processor is configured to determine a fetal lie based on the plurality of detections of the plurality of fetal anatomies, where the output is representative of the fetal lie. In some aspects, the blind sweep protocol may include a vertical sweep and a horizontal sweep, where the processor is configured to determine the fetal spine position only after determination of the fetal lie, where, when the fetal lie is a longitudinal lie, the processor is configured to determine the fetal spine position in the horizontal sweep, where, when the fetal lie is a transverse lie, the processor is configured to determine the fetal spine position using the vertical sweep. In some aspects, the processor is configured to: control the ultrasound probe to perform a calibration sweep before the blind sweep protocol; determine if a length of the calibration sweep is acceptable; prompt a user to rescan when the length of the calibration sweep is not acceptable; and prompt the user to proceed to the blind sweep protocol when the length of the calibration sweep is acceptable. Implementations of the described techniques may include hardware, a method or process, or computer software on a computer-accessible medium.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the fetal orientation detection system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1 is a schematic, diagrammatic representation of an ultrasound imaging system, according to aspects of the present disclosure.
Fig. 2 is a schematic diagram of a processor circuit, according to aspects of the present disclosure.
Fig. 3A is a schematic, diagrammatic, cross-sectional view of a fetus surrounded by amniotic fluid within a uterus accessible through a cervix of a patient, according to aspects of the present disclosure.
Fig. 3B is a schematic, diagrammatic, cross-sectional view of a fetus surrounded by amniotic fluid within a uterus accessible through a cervix of a patient, according to aspects of the present disclosure.
Fig. 3C is a schematic, diagrammatic, cross-sectional view of a fetus surrounded by amniotic fluid within a uterus accessible through a cervix of a patient, according to aspects of the present disclosure.
Fig. 3D is a schematic, diagrammatic, cross-sectional view of a fetus surrounded by amniotic fluid within a uterus accessible through a cervix of a patient, according to aspects of the present disclosure.
Fig. 3E is a schematic, diagrammatic, cross-sectional view of a fetus within a uterus accessible by a cervix, according to aspects of the present disclosure.
Fig. 3F is a schematic, diagrammatic, cross-sectional view of a fetus within a uterus accessible by a cervix, according to aspects of the present disclosure.
Fig. 4 is a schematic, diagrammatic representation of a patient, according to aspects of the present disclosure.
Fig. 5 is a schematic, diagrammatic representation, in hybrid block diagram/flow diagram form, of an example fetal orientation detection system, according to aspects of the present disclosure.
Fig. 6 is a schematic, diagrammatic representation, in hybrid block diagram/flow diagram form, of an example fetal orientation determination method to triage and/or monitor fetal lie and presentation, according to aspects of the present disclosure.
Fig. 7A is a schematic, diagrammatic overview, in block diagram form, of a training mode for an untrained neural network, according to aspects of the present disclosure.
Fig. 7B is a schematic, diagrammatic overview, in block diagram form, of an inference mode or clinical usage mode for the trained neural network, according to aspects of the present disclosure.
Fig. 8 is a schematic, diagrammatic illustration, in block diagram form, of the detection of anatomy (e.g., the placenta, fetal head, fetal spine, etc.), according to aspects of the present disclosure.
Fig. 9 is a schematic, diagrammatic representation, in flow diagram form, of an example fetal orientation determination method, according to aspects of the present disclosure.
Fig. 10 is a schematic, diagrammatic representation, in hybrid flow diagram/block diagram form, of an example fetal orientation determination method, according to aspects of the present disclosure.
Fig. 11 is a schematic, diagrammatic representation, in hybrid block diagram/flow diagram form, of an example 3D/2D spatio-temporal map generation process, in accordance with aspects of the present disclosure.
Fig. 12A is an ultrasound image frame with anatomy detection boxes, including an anatomy detection box for fetal anatomy type A (e.g., the fetal spine), according to aspects of the present disclosure.
Fig. 12B is a fetal spine mask generated from the ultrasound image frame of Fig. 12A, according to aspects of the present disclosure.
Fig. 13A is an ultrasound image frame with anatomy detection boxes, including an anatomy detection box for fetal anatomy type B (e.g., the fetal heart), according to aspects of the present disclosure.
Fig. 13B is a fetal heart mask generated from the ultrasound image frame of Fig. 13A, according to aspects of the present disclosure.
Fig. 14 is a 3D anatomical spatio-temporal map of the fetal anatomy from a vertical sweep (e.g., an M sweep), according to aspects of the present disclosure.
Fig. 15A is a 2D anatomical spatio-temporal map for a vertical sweep (e.g., an M sweep), according to aspects of the present disclosure.
Fig. 15B is a 2D anatomical spatio-temporal map for a horizontal sweep (e.g., a C2 or C3 sweep), according to aspects of the present disclosure.
Fig. 16A-16B is a schematic, diagrammatic representation, in hybrid flow diagram/block diagram form, of an example fetal lie determination method, according to aspects of the present disclosure.
Fig. 17 is a diagram indicating the meaning of different values for the fetal orientation angle θ, according to aspects of the present disclosure.
Fig. 18 is a schematic, diagrammatic representation, of an example fetal orientation determination process, according to aspects of the present disclosure.
Fig. 19 is a 2D anatomical spatio-temporal map for a vertical sweep (e.g., an M sweep), according to aspects of the present disclosure.
Fig. 20 is a schematic, diagrammatic representation, in flow diagram form, of an example fetal lie detection process, according to aspects of the present disclosure.
Fig. 21 is a schematic, diagrammatic representation, of an example fetal orientation determination process, according to aspects of the present disclosure.
Fig. 22 is a schematic, diagrammatic representation of an example fetal orientation determination process, according to aspects of the present disclosure.
Fig. 23 is a schematic, diagrammatic representation, in hybrid flow diagram/block diagram form, of an example fetal lie determination method, according to aspects of the present disclosure.
Fig. 24 is a schematic, diagrammatic representation of an example fetal orientation determination method, according to aspects of the present disclosure.
Fig. 25 is a schematic, diagrammatic representation, in flow diagram form, of an example fetal spine presentation determination method, according to aspects of the present disclosure.
Fig. 26A is a plot of the midpoints of the anatomy detection boxes across a sweep, according to aspects of the present disclosure.
Fig. 26B is a synthesized ultrasound image generated based on the anatomy detection cluster centroids of Fig. 26A, according to aspects of the present disclosure.
Fig. 26C is a schematic, diagrammatic, side cross-sectional view of a fetus inside a uterus of a patient, according to aspects of the present disclosure.
Fig. 27A is a plot of the midpoints of the anatomy detection boxes across a sweep, according to aspects of the present disclosure.
Fig. 27B is a synthesized ultrasound image generated based on the anatomy detection cluster centroids of Fig. 27A, according to aspects of the present disclosure.
Fig. 27C is a schematic, diagrammatic, side cross-sectional view of a fetus inside a uterus of a patient, according to aspects of the present disclosure.
Fig. 28 is a schematic, diagrammatic representation of a sweep acceptance process, according to aspects of the present disclosure.
Fig. 29 is a schematic, diagrammatic representation of a sweep rejection process, according to aspects of the present disclosure.
Fig. 30 is a schematic, diagrammatic representation, in hybrid flow diagram/block diagram form, of an example sweep calibration method, according to aspects of the present disclosure.
Fig. 31 is a schematic, diagrammatic representation of accepted or acceptable calibration sweeps, according to aspects of the present disclosure.
Fig. 32 is a schematic, diagrammatic representation of rejected or unacceptable calibration sweeps, according to aspects of the present disclosure.
Fig. 33 is a schematic, diagrammatic representation of a fetal growth monitoring process, according to aspects of the present disclosure.

### DETAILED DESCRIPTION OF EMBODIMENTS

In accordance with at least one aspect of the present disclosure, a fetal orientation detection system is provided which can identify anatomy of interest for determining fetal orientation (e.g., fetal lie and fetal presentation).

Pregnancy requires constant monitoring by health care providers to avoid conditions that may threaten the lives of the fetus and the mother at birth. For labor management, the diagnosis of fetal presentation may be important to guarantee delivery viability. Knowing the position of the fetus helps a pregnancy care provider determine if it's safe for the patient to have a vaginal delivery or if they should consider a C-section (cesarean delivery).

In this work, the data is acquired from a 3x3 or 5x5 blind sweep protocol, covering the uterus of the mother. From the image sets acquired in these sweeps (also known as cine scans), the disclosed method includes generation of a 3D/2D anatomical spatio-temporal map, requiring only one transverse (M) and one longitudinal scan(C2/C3) to automatically characterize fetus including head and spine position. Ideal position of the fetus is head down, facing the birth parent's back, sometimes known as cephalic or occiput anterior presentation.

Localization of the fetal spine position, along with fetal lie, provides more detailed characterization of the fetus. The positions of the head and spine during the second stage of labor can be practical indicators for predicting the occiput posterior position at delivery. In one study cohort, with occiput posterior and spine anterior position on ultrasound, none of the babies was born in the occiput posterior position. On the other hand, the fetuses presenting occiput posterior position at delivery also had a posterior spine position during the second stage of labor.

The workflow disclosed herein incorporates sweep calibration to ensure the correct capture of relative fetus anatomies during blind protocol, which can lead to more accurate assessment of the fetus' position within the uterus. Additional monitoring components in the workflow will enable a user to keep track of fetus growth and movement over different gestational periods.

The present disclosure provides a method for an end-to-end fetus characterization workflow from blind sweeps, e.g., using low cost ultrasound devices such as Philips Lumify. This method is designed to work on ultrasound scans captured by inexperienced/amateur sonographers (e.g., midwives with a week of training or less), using low-to-moderate quality ultrasound scans. Moreover, currently in a blind protocol, users (and physicians to whom the patient is referred) may not have access to the original ultrasound images. The proposed method provides a summarized view of the fetus inside the uterus through the use of a 2D/3D fetus and/or mother anatomical spatio-temporal map visualization from the captured sweep data. In some aspects, the ultrasound images themselves are not shown to the inexperienced user (who would not have the training to interpret the ultrasound images themselves). The anatomical spatio-temporal map can have different applications to assess the fetus, which gives deeper explainability to the current blind sweep protocol based solution. Additionally, spine localization and sweep calibration steps help to ensure that proper blind scans are performed, and thus improve on current patient outcomes.

Components of the disclosed method include:
Sweep Length Calibration: In this calibration step, the user will perform one longitudinal and one transverse scan to get a rough estimation of where to start and end the scans. In sweep Length Calibration step, user needs to perform a transverse sweep (such as the M sweep of the blind sweep protocol) and a longitudinal sweep (such as the C2 or C3 sweep). Once the sweep is performed, an anatomy detector will detect the anatomies. Once the anatomies are detected, an anatomical spatio-temporal map is generated based on the detections as described below. From the generated map, if the relevant required anatomies (head, heart, abdomen, spine, fetal urinary bladder, femur) are captured in the map, scan positions can be noted for the next blind scan. If the relative anatomies are not captured in the generated map, recalibration/ rescan may be performed. This step ensures the capture of all fetal anatomies for fetal parameter assessment.

Anatomical spatio-temporal map generation: In this step, a 3D/2D map is generated from the selected vertical and horizontal scans (e.g., M and C2 or C3). The map or visualization provides the lie of the fetus inside the uterus. In this step, the input is the bounding box co-ordinates for each relevant anatomy. A mask is generated from bounding box co-ordinates for each individual anatomy. Next, the co-ordinates of each anatomy are extracted from the corresponding mask volume, and a 3D map is generated from the coordinates. The map encompasses relevant anatomies of the fetus as well as of the mother (if required). The map can then be provided to the user as one of the outputs of the workflow. Fetal positional characterization: This block includes two components:
Fetal lie identification: In this step, fetal lie (longitudinal(cephalic/breech)/ transverse/ oblique) is estimated using the generated map.

Fetal spine localization: Spine localization classifies the spine as anterior (spine lies opposite to mother's spine) or posterior (spine lies towards mother's spine). Once the lie of the fetus is identified, the system can select an appropriate scan based on the lie. If the fetus has a longitudinal lie (i.e. in alignment with mother's spine), the system may select a longitudinal scan (e.g., C2 or C3). On the other hand, if the fetus is in a transverse lie, the system may select a transverse scan (e.g., M). The midpoints of the bounding boxes of the heart, amniotic fluid, placenta, and spine are calculated for the selected scan. A 2D plot can then be created from these midpoints to visualize the relative position of the anatomies.

Placenta location estimation can then be done with respect to the amniotic fluid for the selected scan, as described for example in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein. Once the placenta location is determined, the system can assess the relative position of the centroids of the spine and heart clusters with respect to the placenta cluster centroid. If the heart cluster centroid comes in between the placenta and spine cluster centroids, this indicates that the spine location is opposite to the placenta location. Similarly, if the heart centroid cluster doesn't come in between placenta and spine cluster, this indicates that the spine is in alignment with the placenta location.

Monitor: In monitor steps, fetus growth and of fetal movement over different gestational age (GA) can be tracked using the fetal-mother anatomical spatio-temporal map. Growth charts may be used as standard practice with measurements performed using the ultrasound system. One problem is that measurements can typically only be done on a real ultrasound scan, whereas in blind protocol, there is no ultrasound scan showing the anatomies for measurement. Alternately, with the present disclosure, the fetal-maternal anatomy spatio-temporal map can be used to track the growth and monitor fetal movement over the different gestation ages (GAs).

An evaluation of fetal movement is also considered a valuable indicator of fetal health. Over the different GAs, if fetus positional characterization doesn't change, this might be indicative of some abnormality, or may be a precursor to fetal death.

Alternatively, the workflow can be semi-automated, where the user may for example make decision from the anatomical spatio-temporal map. As an alternative, a segmentation model can also be used to get the anatomical masks instead of detection model and bounding boxes.

Anatomy detector: In the current approach, a You Only Look Once (YOLO) deep learning network can be used as an anatomy detector which detects the individual anatomy and provides bounding box coordinates with confidence scores corresponding to each anatomy, as described below.

The present disclosure aids substantially in the capture of high-quality fetal characterizations (e.g., fetal presentation and fetal lie) by minimally trained users, by detecting fetal anatomy, assessing the images to determine fetal orientation, and requiring that the user repeat any sweeps that do not capture the required anatomy. Implemented on a processor in communication with an ultrasound probe, the fetal orientation detection system disclosed herein provides practical improvements in the quality of diagnosis, prophylaxis, and treatment available to patients in underserved areas. This improved imaging methodology transforms a process that is heavily reliant on professional experience into one that is accurate and repeatable even for minimally trained personnel, without the normally routine need to train clinicians such as emergency department personnel to identify fetal orientation from raw ultrasound images. This unconventional approach improves the functioning of the ultrasound imaging system, by providing reliable, repeatable imaging in hospital, office, vehicle, field, and home settings, as well as referral recommendations for patients suspected to have a pregnancy complication such as breech birth.

The fetal orientation detection system may be implemented as a process at least partially viewable on a display, and operated by a control process executing on a processor that accepts user inputs from a keyboard, mouse, or touchscreen interface, and that is in communication with one or more sensor probes. In that regard, the control process performs certain specific operations in response to different inputs or selections made at different times. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the fetal orientation detection system. Certain features may be added, removed, or modified without departing from the spirit of the claimed subject matter.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

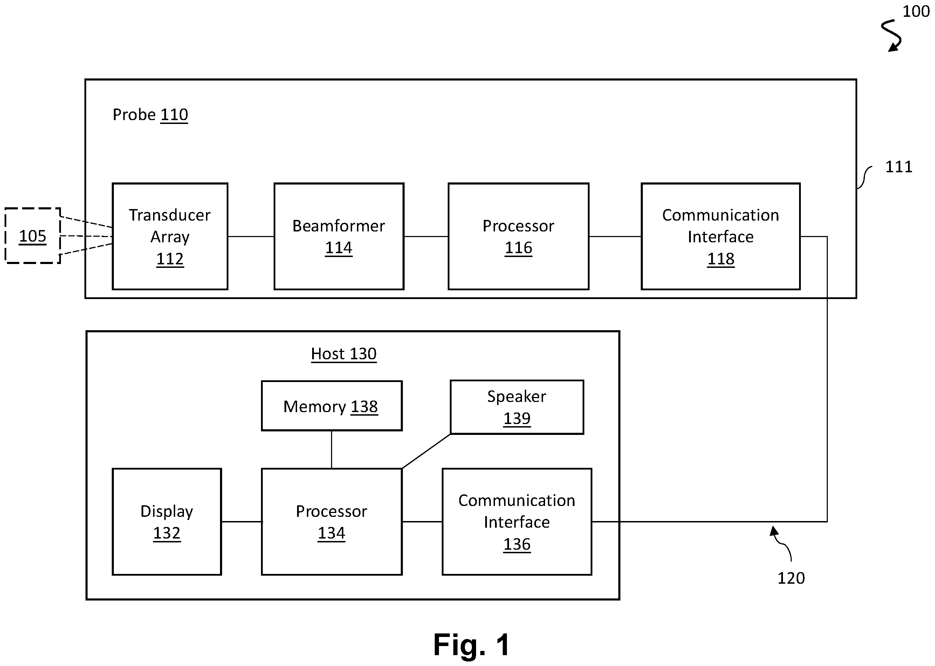

Fig. 1 is a schematic, diagrammatic representation of an ultrasound imaging system 100, according to aspects of the present disclosure. The ultrasound imaging system 100 may for example be used to acquire ultrasound video sweeps, which can then be analyzed by a human clinician or an artificial intelligence to diagnose medical conditions.

The ultrasound imaging system 100 is used for scanning an area or volume of a subject's body. A subject may include a patient of an ultrasound imaging procedure, or any other person, or any suitable living or non-living organism or structure. The ultrasound imaging system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc. In some aspects, the probe 110 may be a curved array probe. Probe 110 may be of any suitable form for any suitable ultrasound imaging application including both external and internal ultrasound imaging.

In some aspects, aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such as a kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, an optional speaker 139, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor circuit 134. The display 132 may be a monitor or any suitable display. The display 132 is configured to display the ultrasound images, image videos, and/or any imaging information of the object 105.

The ultrasound imaging system 100 may be used to assist a sonographer in performing an ultrasound scan. The scan may be performed in a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound imaging system 100 may then analyze the ultrasound image to identify various parameters associated with the acquisition of the image such as the scan window, the probe orientation, the subject position, and/or other parameters. The ultrasound imaging system 100 may then store the image and these associated parameters in the memory 138. At a subsequent imaging procedure, the ultrasound imaging system 100 may retrieve the previously acquired ultrasound image and associated parameters for display to a user which may be used to guide the user of the ultrasound imaging system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, identify and location of anatomical features, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

In some aspects, the host 130 may also include a speaker 180. The speaker 180 may for example be used to provide advisory tones, beeps, or other auditory feedback to the user.

Before continuing, it should be noted that the examples described above are provided for purposes of illustration, and are not intended to be limiting. Other devices and/or device configurations may be utilized to carry out the operations described herein.

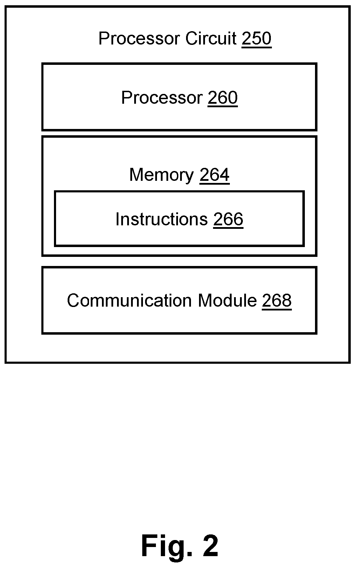

Fig. 2 is a schematic diagram of a processor circuit 250, according to aspects of the present disclosure. The processor circuit 250 may be implemented in the ultrasound imaging system 100, or other devices or workstations (e.g., third-party workstations, network routers, etc.), or on a cloud processor or other remote processing unit, as necessary to implement the method. As shown, the processor circuit 250 may include a processor 260, a memory 264, and a communication module 268. These elements may be in direct or indirect communication with each other, for example via one or more buses.

The processor 260 may include a central processing unit (CPU), a digital signal processor (DSP), a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 260 may also comprise another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 264 includes a non-transitory computer-readable medium. The memory 264 may store instructions 266. The instructions 266 may include instructions that, when executed by the processor 260, cause the processor 260 to perform the operations described herein. Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 250, and other processors or devices. In that regard, the communication module 268 can be an input/output (I/O) device. In some instances, the communication module 268 facilitates direct or indirect communication between various elements of the processor circuit 250 and/or the ultrasound imaging system 100. The communication module 268 may communicate within the processor circuit 250 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, model sharing between the processor and central server, or readings from the ultrasound imaging system 100) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles), 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

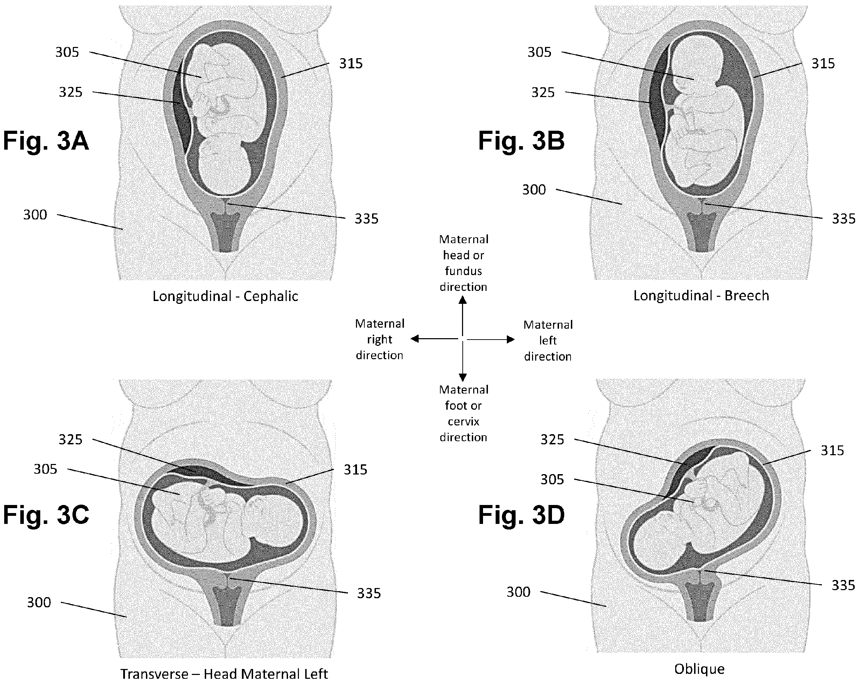

Figs. 3A-3D show a front view of the mother's body.

Fig. 3A is a schematic, diagrammatic, cross-sectional view of a fetus 305 surrounded by amniotic fluid 325 within a uterus 315 accessible through a cervix 335 of a patient 300, according to aspects of the present disclosure.

In describing fetal lie, different sources use different terms to describe how the fetus is positioned inside uterus. Sometimes different sources use terms like fetal lie, fetal presentation, fetal positioning, etc. in different ways and/or interchangeably. For purposes of this document, fetal lie is the axis/dimension along with a majority of the length of the fetus (e.g., crown to rump length) extends; this could also be described axis/dimension that the fetus is orientated relative to the mother's spine (where the mother's spine is taken as kind of a longitudinal reference line). With a longitudinal lie, the length of the fetal body extends along the axis including the maternal head/fundus and maternal foot/cervix directions. Two types of longitudinal lie include cephalic (head down) and breech (head up). With a transverse lie, the length of the fetal body extends along the axis of the maternal right and left directions (e.g., perpendicular, approximately 90 degrees relative to the axis of the maternal head/fundus and maternal foot/cervix directions). Two types of longitudinal lie include head on maternal left (shown in Fig. 3C), head on maternal right (head would be on opposite side of what is shown in Fig. 3C). With an oblique lie, the length of the fetal body extends at oblique angle (non-zero, non-90 degrees) relative to both the maternal fundal-caudal axis and the maternal left-right axis.

In the example shown in Fig. 3A, the fetus is in a longitudinal-cephalic lie. Determining the lie of the fetus is one object of the present disclosure.

Fig. 3B is a schematic, diagrammatic, cross-sectional view of a fetus 305 surrounded by amniotic fluid 325 within a uterus 315 accessible through a cervix 335 of a patient 300, according to aspects of the present disclosure. In the example shown in Fig. 3B, the fetus is in a longitudinal-breech lie. Determining the lie of the fetus is one object of the present disclosure.

Fig. 3C is a schematic, diagrammatic, cross-sectional view of a fetus 305 surrounded by amniotic fluid 325 within a uterus 315 accessible through a cervix 335 of a patient 300, according to aspects of the present disclosure. In the example shown in Fig. 3A, the fetus is in a transverse - head maternal left lie. Determining the lie of the fetus is one object of the present disclosure.
Fig. 3D is a schematic, diagrammatic, cross-sectional view of a fetus 305 surrounded by amniotic fluid 325 within a uterus 315 accessible through a cervix 335 of a patient 300, according to aspects of the present disclosure. In the example shown in Fig. 3A, the fetus is in an oblique lie. Determining the lie of the fetus is one object of the present disclosure.

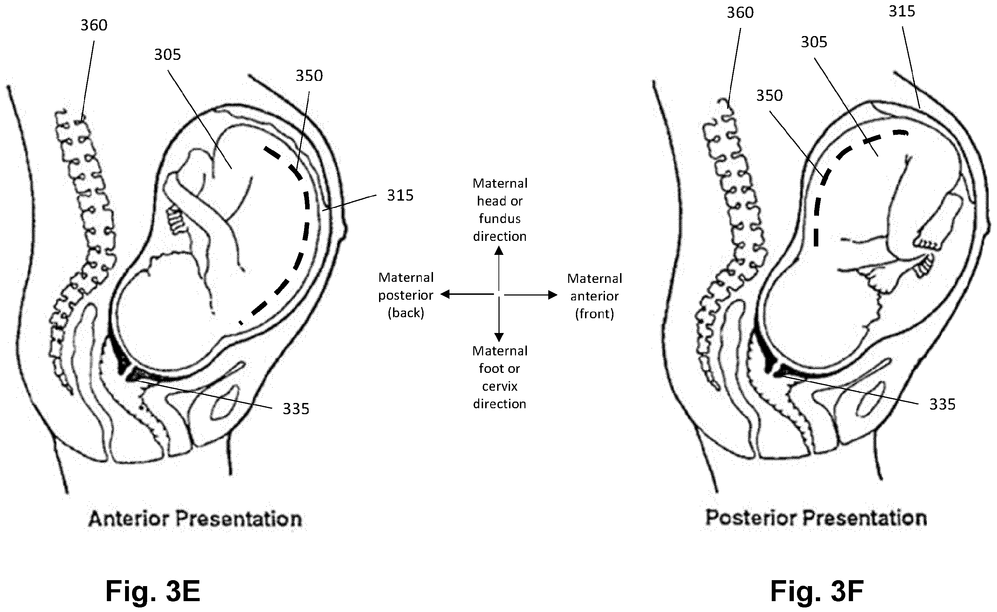

Figs. 3E and 3F are side views of the mother's body (rotated 90 degrees relative to front views shown in Figs. 3A-3D).

Fig. 3E is a schematic, diagrammatic, cross-sectional view of a fetus 305 within a uterus 315 accessible by a cervix 335, according to aspects of the present disclosure.

As described above, different sources use different terms to describe how the fetus is positioned inside uterus. Sometimes different sources use terms like fetal lie, fetal presentation, fetal positioning, etc. in different ways and/or interchangeably. For purposes of this document, fetal presentation refers to the direction the fetus' spine is facing relative to the anterior-posterior axis of the mother. Thus, the maternal posterior (inferior) direction means fetal spine facing toward the maternal back or maternal spine, while the maternal anterior (superior) direction means fetal spine facing toward the maternal front or maternal abdomen/belly (e.g., where the ultrasound probe is positioned to perform ultrasound imaging during the blind sweep protocol).

Thus, the present disclosure describes where the fetus's spine is, in relation to the mother's anatomy (e.g., mother's spine or the mother's abdomen/belly). Other sources describe this as the direction that the fetus' front is facing when inside the uterus (e.g., anterior is facing the mother's spine, while posterior is facing the mother's belly). Thus, fetal presentation includes two types: anterior (fetal spine is positioned proximate to maternal anterior/front; fetal spine is positioned opposite to mother's spine) and posterior (fetal spine is positioned proximate to maternal posterior/back; fetal spine is positioned towards mother's spine).

In the example shown in Fig. 3E, the fetus is in an anterior presentation, with the fetal spine 350 facing away from the maternal spine 360.

Fig. 3F is a schematic, diagrammatic, cross-sectional view of a fetus 305 within a uterus 315 accessible by a cervix 335, according to aspects of the present disclosure. In the example shown in Fig. 3F, the fetus is in a posterior presentation, with the fetal spine 350 facing toward the maternal spine 360.

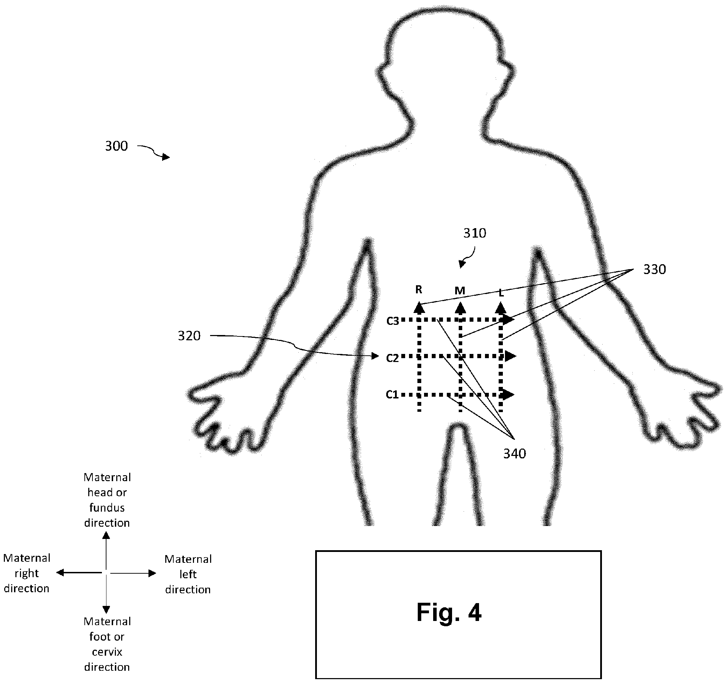

Fig. 4 is a schematic, diagrammatic representation of a patient 300, according to aspects of the present disclosure. Visible on the abdomen 310 of the patient 300 is a desired sweep pattern 320 intended to capture images of desired features of the patient's anatomy. The sweep pattern 320 includes multiple vertical sweep lines 330 and multiple horizontal sweep lines 340. Each sweep line 330, 340 represents a desired path for one imaging sweep of the abdomen 310. In the example shown in Fig. 4, the sweep pattern includes three vertical sweep lines 330 labeled L (patient's left), M (patient's middle), and R (patient's right), all in an upward direction with respect to the patient, and three horizontal sweep lines 340 labeled C1 (bottom), C2 (middle), and C3 (top), all in a right-to-left direction with respect to the patient. However, it is understood that a sweep pattern 320 may include more or fewer sweep lines 330, including vertical sweep lines 330, horizontal sweep lines 330, or combinations thereof, in any combination of upward, downward, left, or right directions, based on the patient's fundal height. For example, if the patient's belly is bigger in size, more sweeps may be needed. Furthermore, a sweep pattern 320 may cover other portions of the patient's body, including but not limited to the head, neck, spine, limbs, etc. Examples of blind sweep protocol include but are not limited to obstetric sweep imaging (OSI), volume sweep imaging (VSI), 6-Stage, Fetal Age Machine Learning Initiative (FAMLI), and Philips.

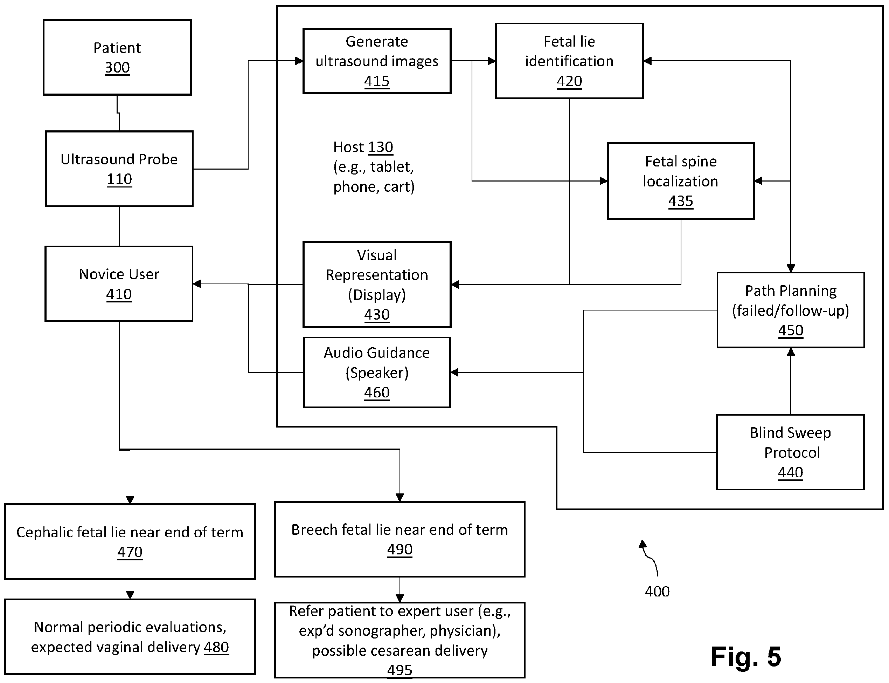

These sweep patterns represent desired probe motion information, including desired positions, a desired velocity or velocities, and/or a desired orientation of the ultrasound probe while the ultrasound probe is obtaining a plurality of ultrasound image frames during the sweep. It is noted that the desired sweep patterns or blind sweep protocols stored in a memory of the processor may include only vertical sweeps, only horizontal sweeps, may include a grid (e.g., 3x3, 5x5, etc.) of vertical and horizontal sweeps, and may also include associated parameters such as desired probe motion (e.g., positions, velocities, and/or orientations) stored in the memory (e.g., blind sweep protocol 440 in Fig. 5). Depending on the implementation, sweeps may include curved, diagonal, and other types of sweeps. The protocols and their associated parameters can for example be based on standards established by authorities in the field (physician organizations, sonographer organizations, etc.), published in scholarly journals/textbooks, etc.

Fig. 5 is a schematic, diagrammatic representation, in hybrid block diagram/flow diagram form, of an example fetal orientation detection system 400, according to aspects of the present disclosure. An ultrasound probe 110 operated by a novice user 410 performs a blind sweep protocol 440 on the body of a patient 300 and sends ultrasound imaging data to a host 130, such as a tablet, smartphone, ultrasound cart, etc. In step 415, the host 130 generates ultrasound images using the ultrasound image data obtained by the ultrasound probe 110. The host 130 can control the ultrasound probe 110 to obtain the ultrasound image data (e.g., the host 130 establishes communication with the ultrasound probe 110, the host 130 sends control signals to start and/or stop acquisition of ultrasound image data, the host 130 sends power signals to power the ultrasound probe 110, etc.).

In step 420, the host 130 uses the ultrasound images generated in step 415 and/or the ultrasound image data used to generate the ultrasound images to determine the fetal lie, as described in more detail below. In step 435, the host 130 uses the ultrasound images generated in step 415 and/or the ultrasound image data used to generate the ultrasound images to determine the location of the fetal spine, as described in more detail below. In step 430, the host generates a visual representation and outputs the visual representation on a display (e.g., display 132 of Fig. 1), showing the fetal lie and/or fetal presentation.

In step 450, the host 130 determines whether the planned path of the blind sweep protocol 440 has been followed adequately. If no, execution proceeds to steps 430 and 460, wherein the host 130 provides feedback (e.g., audio feedback through a speaker and/or visual feedback from a display) to repeat one or multiple sweeps of the blind sweep protocol 440. In some aspects, the determination in step 450 can be based on the results of analysis in step 420 of the fetal lie and/or in step 435 of the fetal spine's location. For example, if there is insufficient ultrasound image data to perform the determinations in step 420 and/or step 435 or if the ultrasound image data that has been obtained is not suitable to perform the determinations in step 420 and/or step 435, then step 450 can determine that the one or multiple sweeps of the blind sweep protocol 440 has not been completed correctly. The one or multiple sweeps that need to be performed again can be communicated to the user through visual or audio feedback in steps 430 and 460.

In some aspects, the planned path determined in step 450 of the blind sweep protocol 440 can be used by the host 130 to determine the fetal lie in step 420 and/or the location of the fetal spine in step 435. For example, the planned path determined in step 450 can provide absolute or relative spatial information (e.g., vertical location, horizontal location) about the ultrasound image data obtained by the ultrasound probe and/or the ultrasound images generated by the host. For example, referring again to Fig. 4, the planned path determined in step 450 can indicate that the M sweep is horizontally located (left-right direction in Fig. 4) between the R sweep and the L sweep. The host 130 can use this spatial information from step 450 to process the ultrasound image data and/or ultrasound images to determine the fetal lie in step 420 and/or the location of the fetal spine in step 435.

Both fetal lie and fetal spine location can provide helpful information related delivery. For example, a cephalic (head down) lie with, fetal spine anterior presentation (baby facing mother's spine) may be ideal for vaginal delivery. Conversely, a cephalic (head down), fetal spine posterior (baby facing mother's belly) may be less ideal, but vaginal delivery may still be possible, whereas with a breech (head up) like, cesarean delivery will likely be considered.

Based on the visual representation 430, the novice user 410 makes a triage decision to either rule out or rule in a breech fetal lie. In step 470, the novice user 410 rules out breech lie (e.g., determines that the fetus is in a cephalic lie), and in step 480, the novice user 410 performs or recommends normal periodic evaluations for the patient 300. In step 490, the novice user 410 rules in breech fetal lie, and in step 495, the novice user 410 refers the patient to an expert user such as an experienced sonographer, radiologist, obstetrician, or other physician for follow-up imaging and diagnosis.

Flow diagrams and block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available. Similarly, block diagrams may show a particular arrangement of components, modules, services, steps, processes, or layers, resulting in a particular data flow. It is understood that some aspects of the systems disclosed herein may include additional components, that some components shown may be absent from some aspects, and that the arrangement of components may be different than shown, resulting in different data flows while still performing the methods described herein. The logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein. For example, to provide an assessment of the fetal lie and/or fetal presentation, the system may need to generate the visual representation 430 within one second of completing the blind sweep protocol.

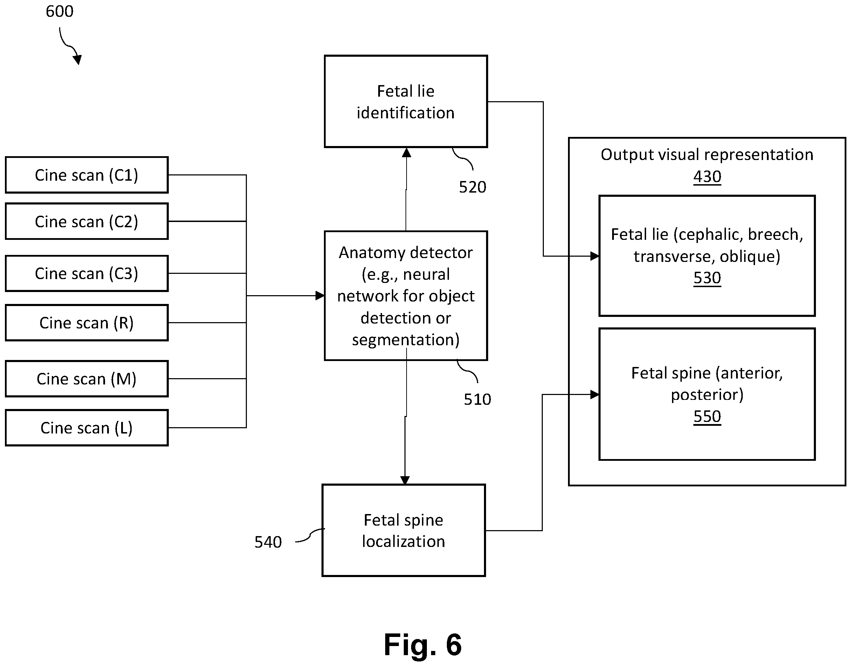

Fig. 6 is a schematic, diagrammatic representation, in hybrid block diagram/flow diagram form, of an example fetal orientation determination method 600 to triage and/or monitor fetal lie and presentation, according to aspects of the present disclosure. Images from a sweep of the blind sweep protocol can be organized into an image sequence known as a cine scan or cineloop (e.g., multiple image frames making up a video segment). The host thus receives or assembles cine scans C1, C2, C3, R, M, and L, corresponding to the blind abdominal sweeps C1, C2, C3, R, M, and L shown in Fig. 6.

In step 510, these cine scans are fed into an object detector (e.g., a trained neural network) to detect the presence of and, if present, the location of the anatomical region of interest (e.g., fetal head, fetal spine, etc.) in each ultrasound image frame of the cine scan. Depending on the implementation, execution then proceeds to either or both of steps 520 or 540.

In step 520, the method includes determining (e.g., using heuristics and/or a trained classifier) the fetal lie within the uterus, and generating an output visual representation 430 that includes a visual or text representation of the fetal lie 530.

In step 540, the method includes (e.g., using a heuristic anomaly detector and/or a trained classifier) assessing whether the fetal spine is in an anterior or posterior presentation, and generating an output visual representation 550 indicative of the fetal spine presentation.

Depending on the implementation, the visual representation 430 can include text labels or images/graphics (including both not limited to the images/graphics described herein), and/or combinations or thereof. The anatomy detector 510 can identify fetal anatomy (fetal head, fetal spine, fetal heart, fetal abdomen, fetal urinary bladder) and/or maternal anatomy (placenta, amniotic fluid, maternal spine, etc.). Inputs to the anatomy detector 510 are ultrasound image frames from horizontal sweep(s) and/or vertical sweep(s). For example, each sweep can be a cine scan (e.g., an ultrasound video made up of multiple ultrasound image frames). The output of the anatomy detector 510 can depend on the type of predictive model/network being used. For an objection detection neural network, the output may include one or more bounding boxes identifying anatomy. For a segmentation neural network, the output may include the contour or perimeter of the anatomy.

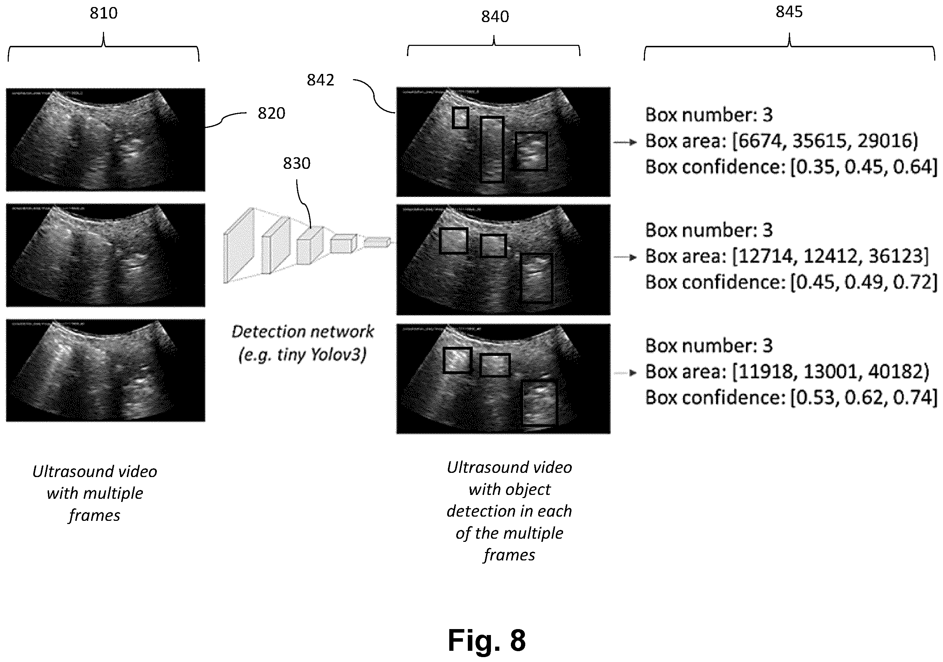

The anatomy detector may use available networks for object detection (e.g., YOLO, R-CNN, R-FCN, or otherwise as described below in Fig. 8).

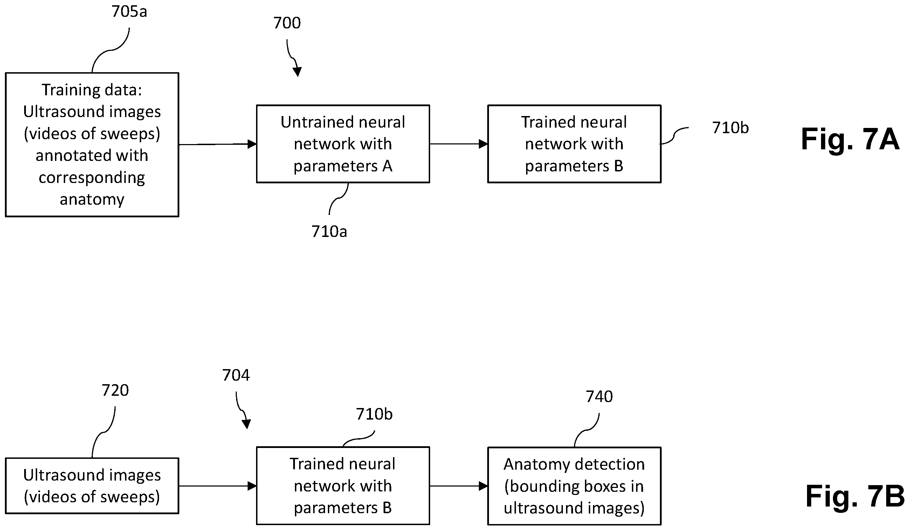

Fig. 7A is a schematic, diagrammatic overview, in block diagram form, of a training mode 700 for an untrained neural network 710a, according to aspects of the present disclosure. In the example shown in Fig. 7A, a set of training data 705a includes ultrasound cineloops of probe sweeps annotated with the corresponding anatomy (placenta, fetal head, fetal spine, etc.). The training data 705a is fed into an untrained neural network 710a in an iterative training process that will be familiar to a person of ordinary skill in the art.

The parameters of a network model (e.g., the weights at each artificial neuron) are initialized with initial values A that may be random values or with results from training on prior datasets. In an iterative process, the network is used to make detection inferences on the training images, the results are compared with the ground truth annotations, and an optimizer is used to adjust the network parameters B until a metric of accuracy is maximized.

Thus, an output of this training process 700 is a trained neural network 710b, wherein the parameters B (e.g., weights) are optimized for generating accurate bounding boxes for the anatomy imaged in the training data 705a.

Fig. 7B is a schematic, diagrammatic overview, in block diagram form, of an inference mode or clinical usage mode 704 for the trained neural network 710b, according to aspects of the present disclosure. In clinical usage, an ultrasound video, cineloop, or cine sweep 720 of the blind sweep is fed to the trained and validated neural network 710b for analysis. The trained and validated neural network 710b then produces, as an output, anatomy detection bounding boxes 740 for each image (or the entire sweep). In some aspects, a confidence value can be determined as a normalized value in the range [0-1], where 0 indicates lowest confidence, and 1 indicates highest confidence that the detection is correct.
Fig. 8 is a schematic, diagrammatic illustration, in block diagram form, of the detection of anatomy (e.g., the placenta, fetal head, fetal spine, etc.), according to aspects of the present disclosure. A cineloop 810 comprising multiple frames 820 is fed into a trained object detector 830.

The object detector 830 may implement or include any suitable type of learning network. For example, in some aspects, the object detector 830 could include a neural network, such as a convolutional neural network (CNN). In addition, the convolutional neural network may additionally or alternatively be an encoder-decoder type network, or may utilize a backbone architecture based on other types of neural networks, such as an object detection network, classification network, etc. One example backbone network is the Darknet YOLO backbone, (e.g., Yolov3) which can be used for object detection. The CNN may for example include a set of N convolutional layers, where N may be any positive integer. Fully connected layers can be omitted when the CNN is a backbone. The CNN may also include max pooling layers and/or activation layers. Each convolutional layer may include a set of filters configured to extract features from an input (e.g., from a frame of the ultrasound video). The value N and the size of the filters may vary depending on the aspects. In some instances, the convolutional layers may utilize any non-linear activation function, such as for example a leaky rectified non-linear (ReLU) activation function and/or batch normalization. The max pooling layers gradually shrink the high-dimensional output to a dimension of the desired result (e.g., bounding boxes of a detected feature). Outputs of detection network may include numerous bounding boxes, with most having very low confidence scores and thus being filtered out or ignored. Fully connected layers may be referred to as perception or perceptive layers. In some aspects, perception/perceptive and/or fully connected layers may be found in object detector 830 (e.g., a multi-layer perceptron).

These descriptions are included for exemplary purposes; a person of ordinary skill in the art will appreciate that other types of learning models, with features similar to or dissimilar to those described above, may be used instead or in addition, without departing from the spirit of the present disclosure.

Outputs of the object detector 830 may include an annotated cineloop 840 made up of a plurality of annotated image frames 842, possibly including per-frame metrics 845 such as the confidence level of the detections.

The systems and methods disclosed herein are broadly applicable to different types of features, and can for example draw boxes around the placenta, fetal spine, fetal head, or other anatomical features depending on the implementation. The object detector can be one class or multi-class, depending how the model is built. If another detector is trained separately, then both models can be run separately (e.g., one model for each feature type). Otherwise, multiple feature classes can be identified, and enclosed in detection boxes, at the same time. The ML model for placenta detection can use exactly the same structure as a model for cervix detection. One can either train/run a single detector that detects multiple feature types (a "multi-class detector") and provides their locations as an output, along with the confidence score and feature type (class) of each detection. Alternatively, one could run several "single-class" detectors, each trained to detect a single feature type/class. These separate single-class detectors may in some cases have the same architecture (e.g., layers and connections), but may have been trained with different data (e.g., different images and/or annotations).

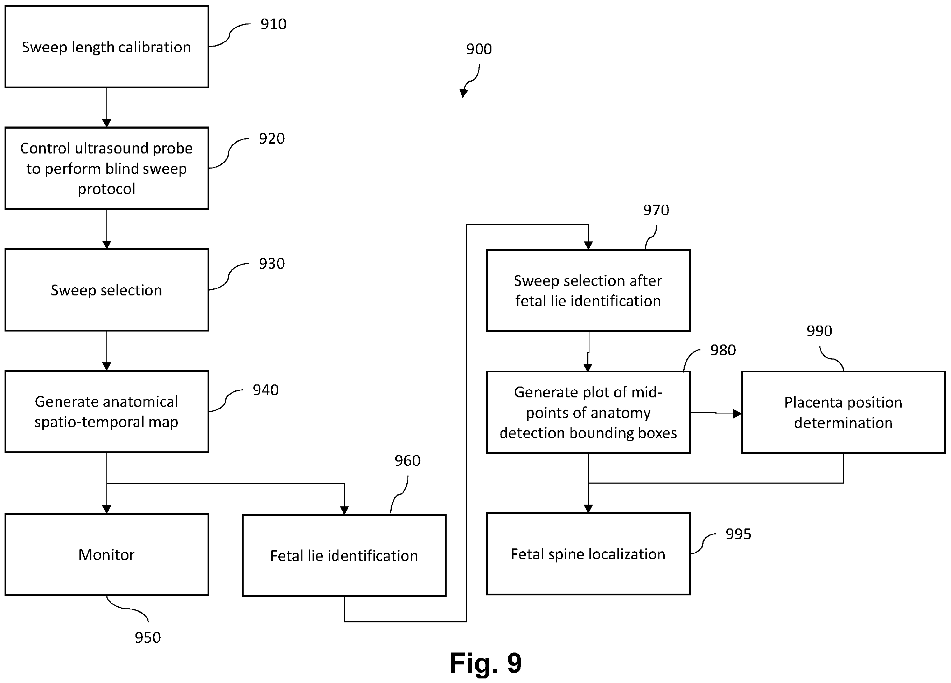

Fig. 9 is a schematic, diagrammatic representation, in flow diagram form, of an example fetal orientation determination method 900, according to aspects of the present disclosure.

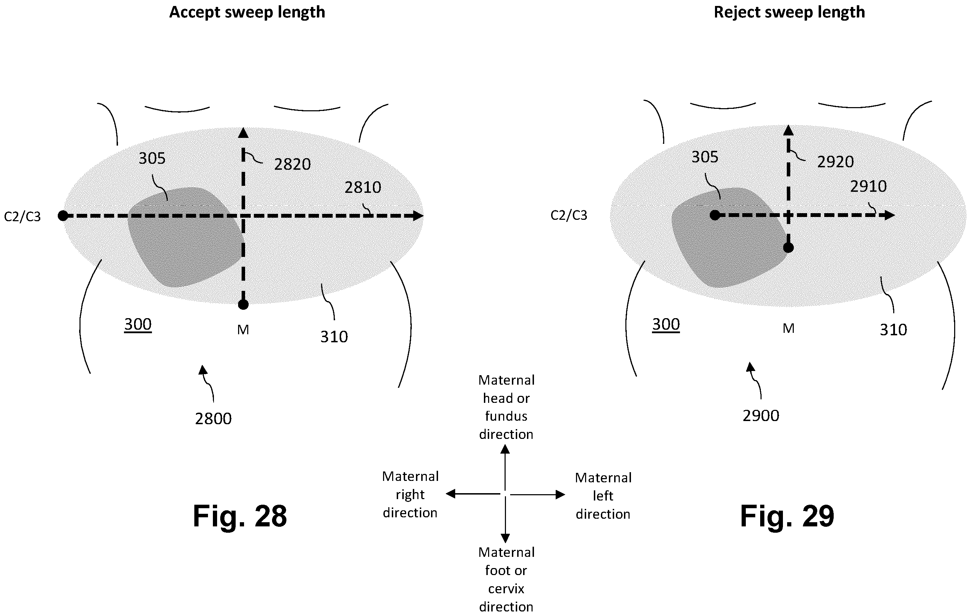

In step 910, the method 900 includes a sweep length calibration step, as described in detail in Figs. 28-31, below. Execution then proceeds to step 920.

In step 920, the method 900 includes controlling the ultrasound probe to perform the blind sweep protocol, as described in Fig. 4, above. Execution then proceeds to step 930.

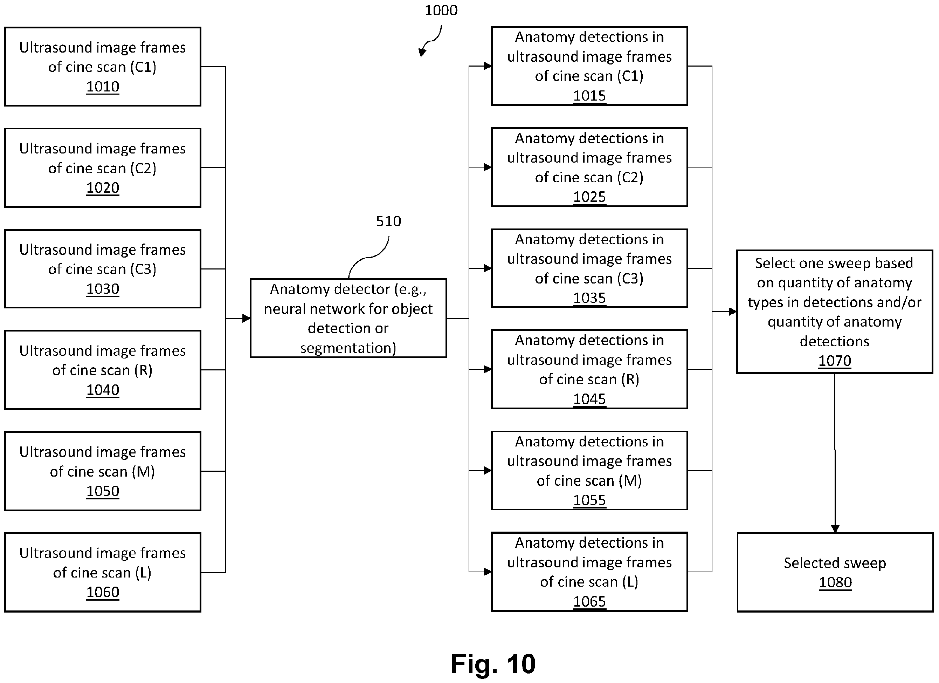

In step 930, the method 900 includes selecting a sweep from the blind sweep protocol for determining fetal lie and fetal presentation, as described in detail in Fig. 10, below. Execution then proceeds to step 940.

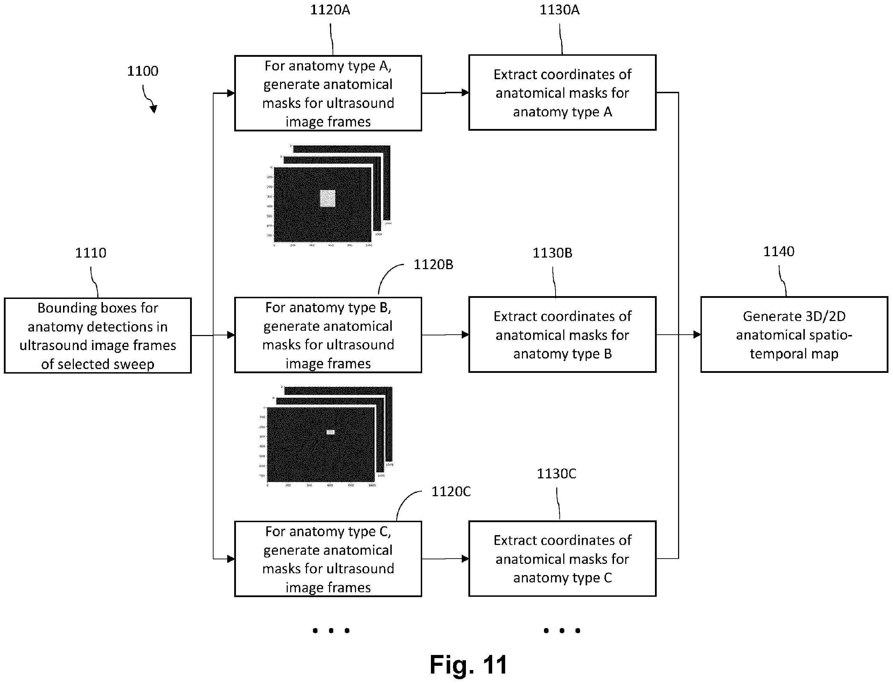

In step 940, the method 900 includes generating a spatio-temporal map, as described below in Figs. 11-15B. Execution then proceeds to steps 950 and 960.

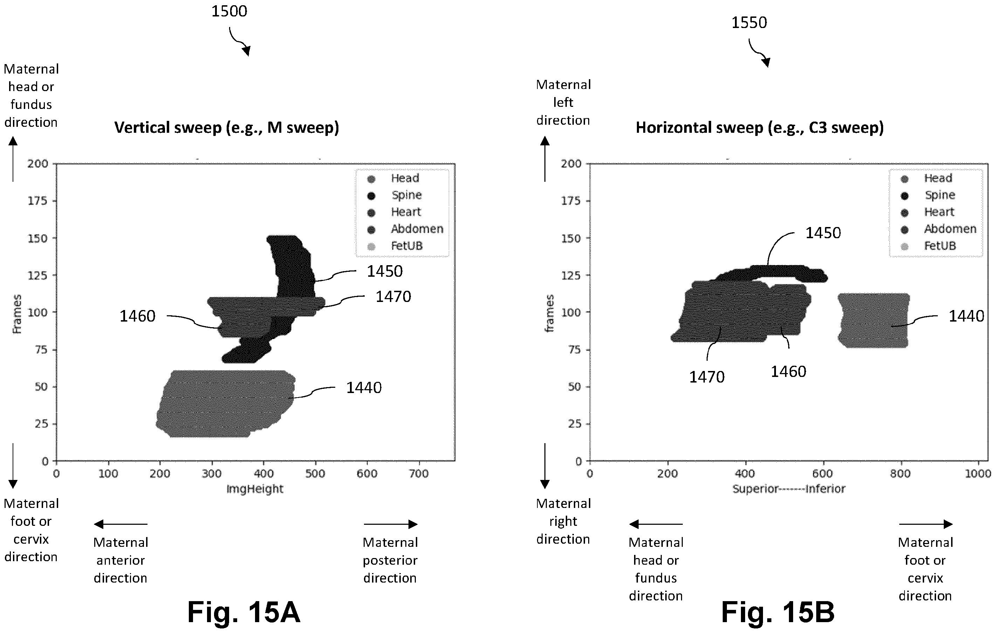

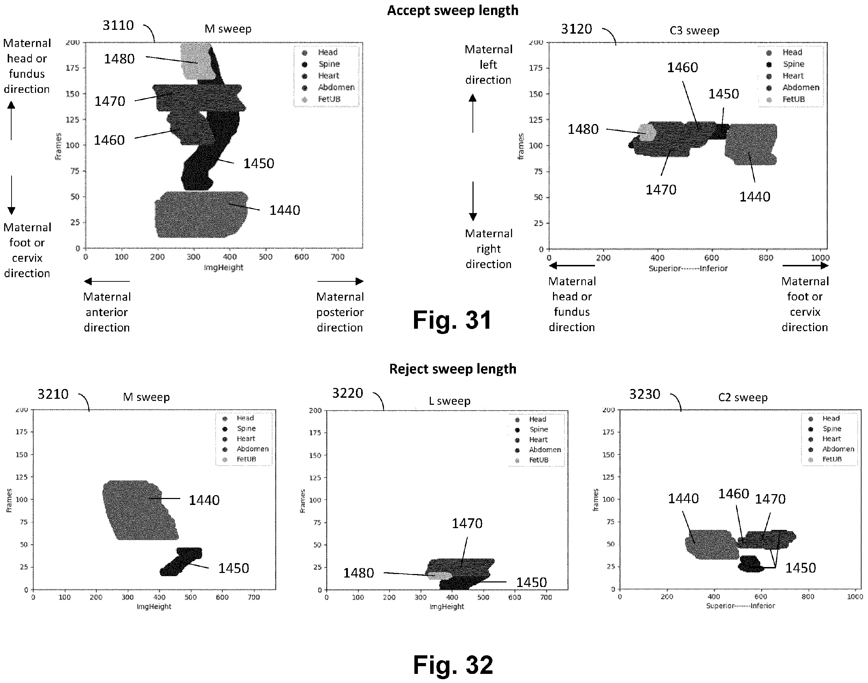

In step 950, the method includes monitoring the growth and movement of the fetus across different gestational ages, as described for example in Fig. 32, below. The method 900 is now complete. In step 960, the method 900 includes determining the fetal lie, as described in detail in Figs. 15-23, below. Execution then proceeds to step 970.

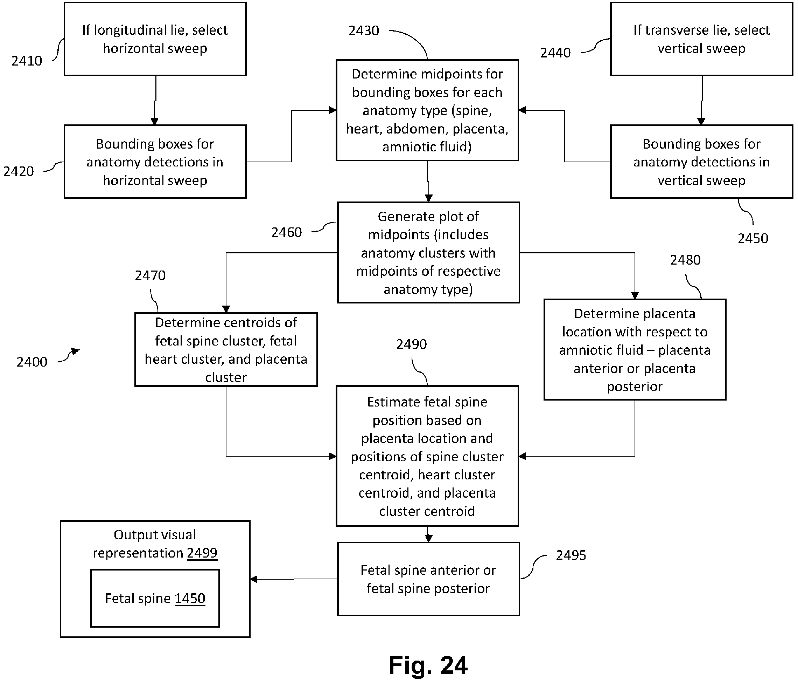

In step 970, the method 900 includes selecting a sweep after fetal lie identification, as described in detail in Fig. 24, below. Execution then proceeds to step 980.

In step 980, the method 900 includes generating a plot of the midpoints of the anatomy detection bounding boxes, as described in detail in Figs. 24-27C. Execution then proceeds to steps 995 or, optionally, to step 990.

In step 990, the method 900 includes determining the position of the placenta, as described for example in Fig. 24, below, and in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein. Execution then proceeds to step 995.

In step 995, the method 900 includes localizing the fetal spine, as described in detail in Fig. 24-27C, below. The method 900 is now complete.

Fig. 10 is a schematic, diagrammatic representation, in hybrid flow diagram / bloc diagram form, of an example fetal orientation determination method 1000, according to aspects of the present disclosure. Ultrasound image frames 1010, 1020, 1030, 1040, 1050, and 1060 (from scans C1, C2, C3, R, M, and L, respectively) are fed through the object detector 510 to yield anatomy detections 1015, 1025, 1035, 1045, 1055, and 1065 for scans C1, C2, C3, R, M, and L, respectively.

To obtain the image frames 1010-1060, the system must control the ultrasound probe to perform the blind sweep protocol, including multiple sweeps (e.g., multiple horizontal sweeps, multiple vertical sweeps). Selection of one sweep among multiple sweeps of the blind sweep protocol may for example be done to reduce computational complexity and/or speed up processing time. In some aspects, the system may select multiple sweeps (though less than all sweeps of blind sweep protocol). In still other aspects (e.g., where sufficient computing power and memory are available), the system may use all sweeps.

One example of selecting one sweep uses a count-based determination - based on a quantity of anatomy types in detections (e.g., which sweep has the most anatomy types) and/or the quantity of detections (e.g., total detections and/or detections of particular anatomy types). Depending on the implementation, all sweeps can be considered or can be hierarchical/preferential. For example, the system can prefer vertical sweeps, and may select a horizontal sweep only if no acceptable vertical sweep is available.

In an example, the system starts with the M sweep, and determines if the quantity of anatomy types and/or quantity of detections in that sweep satisfies certain threshold value(s). If so, the system may choose the M sweep. However, if the threshold value(s) are not satisfied, then the system may consider the L sweep and R sweep, and may choose whichever one has the greater quantity of anatomy types and/or quantity of detections.

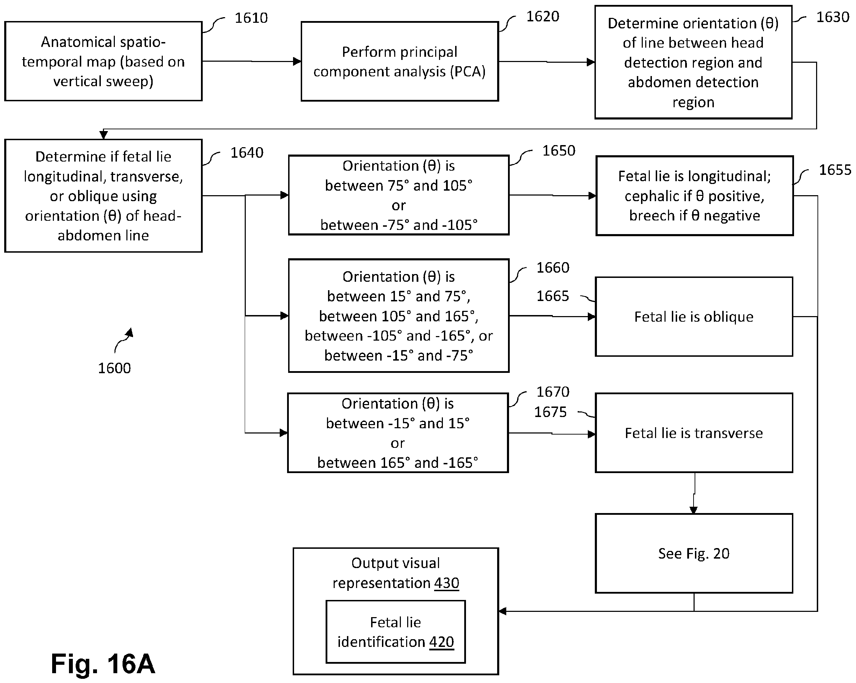

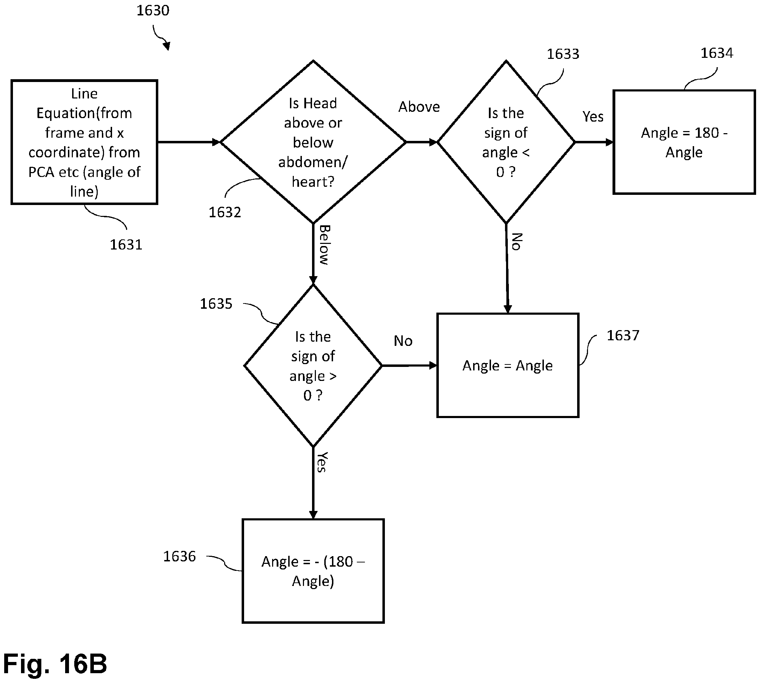

Based on experimental data, it is expected that a vertical sweep (in particular, the middle vertical sweep or M sweep) is going to be the most frequently selected, because experimental data shows that vertical sweeps (e.g., the M sweep) usually have the most anatomy types and/or detections (regardless of which of fetal lie). However, it could be any sweep that is selected and, as describe in Fig. 16, the system may in fact need to use both a vertical sweep and a horizontal sweep to construct the 3D/2D spatio-temporal model.

Fig. 11 is a schematic, diagrammatic representation, in hybrid block diagram / flow diagram form, of an example 3D/2D spatio-temporal map generation method 1100, in accordance with aspects of the present disclosure.

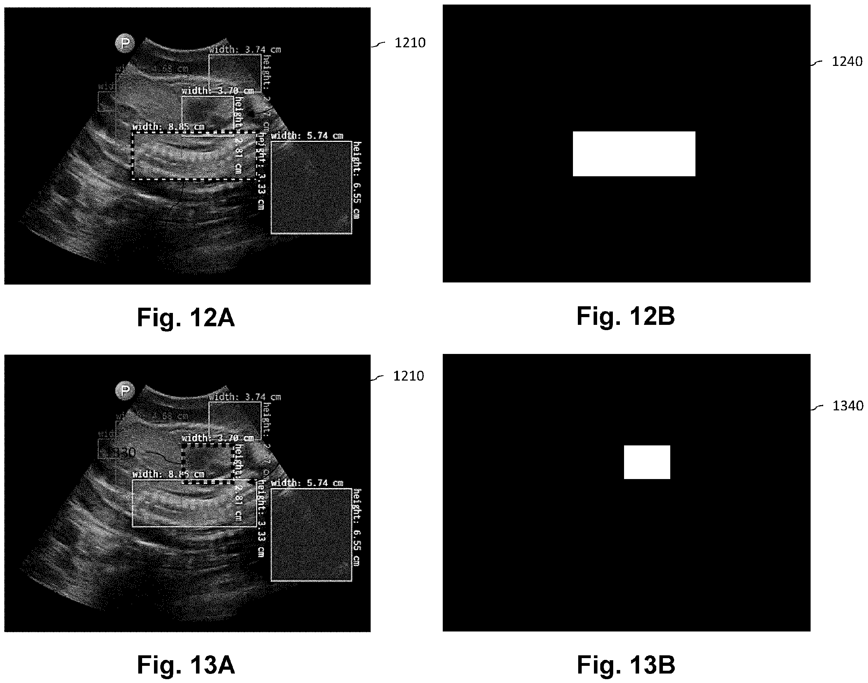

In steps 1120A 1120B, and 1120C, etc., the method 1100 includes receiving the bounding boxes 1110 for anatomy detections in the ultrasound image frames of the selected sweep, and generating masks that cover the respective bounding boxes for anatomy type A, type B, type C, etc., as shown below in Figs. 12A-13B. Execution then proceeds to steps 1130A, 1130B, 1130C, etc.

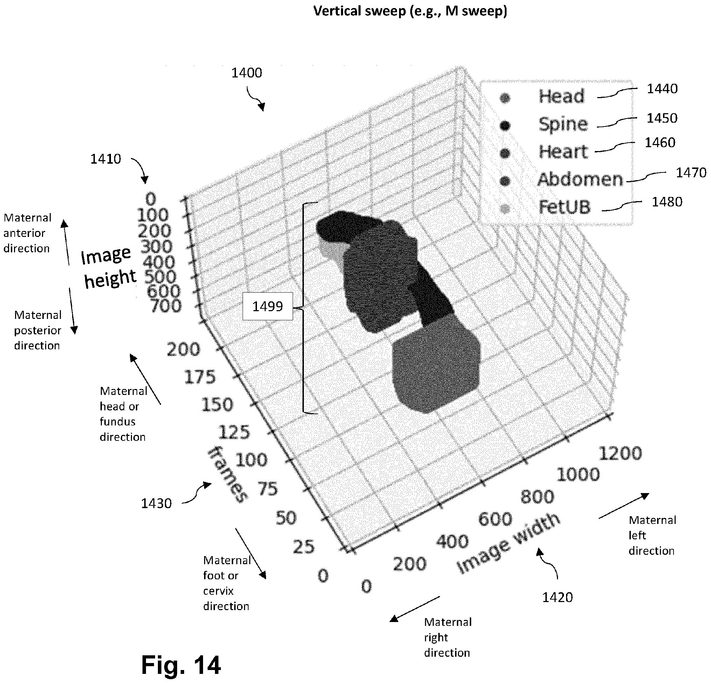

In steps 1130A, 1130B, 1130C, etc., the method 1100 includes extracting coordinates of the anatomical masks for anatomy type A, type B, type C, etc. Execution then proceeds to step 1140. In step 1140, the method 1100 includes generating a 3D anatomical spatio-temporal map, by placing each mask in an appropriate location based on its frame number and position within the frame, as shown below in Fig. 14. The method 1100 is now complete.

Thus, the method 1100 starts with the output of the anatomy detector (e.g., a neural network for object detection). In an example, the selected sweep includes 200 frames. Some frames could have no detections, some frames could have detections of only anatomy type A, some frames could have detections of only anatomy type B, some frames could have detections of both anatomy types A and B, etc.. However, in the example, 80 of those frames have detections of anatomy type A (e.g., fetal spine), 70 of those frames have detection of anatomy type B (e.g., fetal heart) and so on. The same 200 frames from the selected sweep are then provided to the mask generation step 1130A for anatomy type A, mask generation step 1130B for anatomy type B, etc. This means that, for a single ultrasound image frame, multiple masks can be generated.

The same frames are processed differently because mask generation for anatomy type A is focusing on detections of anatomy type A, mask generation for anatomy type B is focusing on detections of anatomy type B. This is described more in Figs. 12B and 13B. In the example, the mask is binary - some pixels/area have a value of 1, and some pixels/area have a value of 0.

Fig. 12A is an ultrasound image frame 1210 with anatomy detection boxes 1220, including an anatomy detection box 1230 for fetal anatomy type A (e.g., the fetal spine), according to aspects of the present disclosure. Each bounding box 1220 is an output of the anatomy detector and represents a different anatomy, such as the head, heart, abdomen, etc. Locations of the bounding boxes can be used to generate masks, which are then used to generate the 3D spatio-temporal anatomy map.

Fig. 12B is a fetal spine mask 1240 generated from the ultrasound image frame 1210 of Fig. 12A, according to aspects of the present disclosure. The mask 1240 includes a "white" or "1" portion 1250 and a "black" or "0" portion 1260. The white portion 1250 fully and exclusively covers the fetal spine bounding box 1230 of Fig. 12A, such that each pixel located within the bounding box 1230 is colored white (e.g., a numerical value of 1), and each pixel located outside of the bounding box 1230 is colored black (e.g., a numerical value of 0). This is repeated for all ultrasound images frames of the selected sweep and for all anatomy types. The white portions 1250 of successive masks in a sweep can then be used to create the "spine" portion of a 3D spatio-temporal map, as shown below in Fig. 14.

The coordinates of the white rectangle 1250 are the same as those of the corresponding bounding box 1230 in Fig. 12A. As described in more detail in Figs. 14 and 15, the x dimension and y dimension of the white portion 1250 will depend on whether the ultrasound image frame is from a vertical sweep or a horizontal sweep. The size and location of the white portion also depends on the size and location of the fetal spine in each successive image frame of the sweep. Frames that do not include the fetal spine will not have a white portion 1250 for the mask 1240 (e.g., the mask 1240 will be entirely black).

Fig. 13A is an ultrasound image frame 1210 with anatomy detection boxes 1220, including an anatomy detection box 1330 for fetal anatomy type B (e.g., the fetal heart), according to aspects of the present disclosure. Each bounding box 1220 is an output of the anatomy detector and represents a different anatomy, such as the head, heart, abdomen, etc. Locations of the bounding boxes can be used to generate masks, which are then used to generate the 3D spatio-temporal anatomy map.

Fig. 13B is a fetal heart mask 1340 generated from the ultrasound image frame 1210 of Fig. 13A, according to aspects of the present disclosure. The mask 1340 includes a "white" or "1" portion 1350 and a "black" or "0" portion 1360. The white portion 1350 fully and exclusively covers the fetal heart bounding box 1330 of Fig. 13A, such that each pixel located within the bounding box 1330 is colored white (e.g., a numerical value of 1), and each pixel located outside of the bounding box 1330 is colored black (e.g., a numerical value of 0). The white portions 1350 of successive masks in a sweep can then be used to create the "heart" portion of a 3D spatio-temporal map, as shown below in Fig. 14.

Fig. 14 is a 3D anatomical spatio-temporal map 1400 of the fetal anatomy from a vertical sweep (e.g., an M sweep), according to aspects of the present disclosure. The spatio-temporal map includes three coordinate axes: the image height axis 1410 (spanning the maternal anterior-posterior axis), image width axis 1420 (spanning the material left-right axis), and frame number 1430 (spanning the maternal cranial-caudal axis or head-foot axis). Anatomy detections 1499 are filled in by adding each bounding box (e.g., the white portions of each mask) for each anatomy type to each frame number 1430 of the map. In the example shown in Fig. 14, frame number 100 contains a rectangle representing a detection mask or bounding box representing the fetal head 1440, whereas frame number 150 contains rectangles representing detection masks or bounding boxes for the abdomen 1470, heart 1460, and spine 1450, and frame number 175 contains rectangles representing the fetal urinary bladder 1480 and spine 1450. Thus, by summing the masks for each anatomy type and frame number, the 3D anatomical spatio-temporal map 1400 generates a 3D representation of the locations of fetal anatomy within the patient's body, which can then be used to calculate the fetal lie and/or fetal presentation. For example, in the example shown in Fig. 14, the fetus is in a longitudinal-cranial lie (see Fig. 3A), with anterior fetal spine presentation (see Fig. 3E). However, the 3D spatio-temporal map 1400 does not include details that may be visible in a raw ultrasound image, such as the sex of the fetus.

In some aspects, a 2D projection of the 3D spatio-temporal map 1400 can be displayed to the user. The system may include a user input to rotate the map 1400 to different viewing angles, and/or a user input to turn on/off anatomy detection regions (e.g., turn off placenta region and amniotic fluid region if user only wants to see fetal anatomies).

Fig. 15A is a 2D anatomical spatio-temporal map 1500 for a vertical sweep (e.g., an M sweep), according to aspects of the present disclosure. In the example shown in Fig. 15A, the 2D anatomical spatio-temporal map 1500 is a projection or cross-section of the 3D anatomical spatio-temporal map 1400 of Fig. 14, in the frame number / image height plane. Visible are the fetal head 1440, fetal spine 1450, fetal heart 1460, and fetal abdomen 1470, in a longitudinal, cephalic lie (see Fig. 3A).

Fig. 15B is a 2D anatomical spatio-temporal map 1500 for a horizontal sweep (e.g., a C2 or C3 sweep), according to aspects of the present disclosure. In the example shown in Fig. 15B, the 2D anatomical spatio-temporal map 1500 is a projection or cross-section of the 3D anatomical spatio-temporal map 1400 of Fig. 14, in the frame number / maternal head/foot plane. Visible are the fetal head 1440, fetal spine 1450, fetal heart 1460, and fetal abdomen 1470, in a longitudinal, cephalic lie (see Fig. 3A).

Figs. 15A and 15B can be representative of the same patient. The 2D maps are 2D planes/slices or projections of a 3D map; what's on the x axis and y axis will depend on whether the sweep is vertical or horizontal, and on where the 2D slice is taken from the 3D map.

Fig. 16A is a schematic, diagrammatic representation, in hybrid flow diagram/block diagram form, of an example fetal lie determination method 1600, according to aspects of the present disclosure.

In step 1620, the method 1600 includes performing principal component analysis (PCA) on an anatomical spatio-temporal map 1610 (e.g., based on a vertical sweep). In an example, inputs to the PCA include spine frame coordinates and spine midpoints along the x axis (image width i.e. maternal left, right). The PCA then outputs a straight line which fits the maximum variation along the path. PC1 will give the spread along frame axis. Similarly, PC2 and other components will give spread in the other axes. Next, the slope of the line is calculated, which yields the value of theta. Execution then proceeds to step 1630.

In step 1630, the method 1600 includes determining the orientation of a line drawn between the head detection region and the abdomen detection region (e.g., between the centers of the two regions). In some aspects, the method 1600 instead uses a line determined by the PCA, or a line drawn between the fetal abdomen and the fetal heart. Execution then proceeds to step 1640. Step 1630 is shown in more detail in Fig. 16B.

It is noted that in Fig. 16A, orientation is described as the orientation of the longest component. In other figures, below, the PCA line is not always the one that is used to determine theta. Rather, it may be the line between head detection region and the abdomen region that is used for the orientation (θ). Different approaches may be used to give more accurate results. Considering a straight line fit of two points (abdomen and head cluster midpoint), may give more accurate results and perform better for different edge cases, such as when the fetal head is not aligned with the body, but lying in a curvilinear fashion.

In step 1640, the method 1600 includes determining if the fetal lie is longitudinal, transverse, or oblique, using the orientation θ of the head-abdomen line. Whether head is below or above the abdomen /heart cluster based on that we can decide the lie. Execution then proceeds to step 1650, 1660, or 1670, depending on the value of θ.

In step 1650, the method 1600 includes determining that the orientation (Θ) is between 75° and 105° or between -75° and -105°. Execution then proceeds to step 1655.

In step 1655, the method 1600 includes determining that the fetal lie is longitudinal, and either cephalic if θ is positive, or breech if θ is negative. Execution then proceeds to step 430.

In step 1660, the method 1600 includes determining that the orientation (Θ) is between 15° and 75°, between 105° and 165°, between -105° and -165°, or between -15° and -75°. Execution then proceeds to step 1665.

In step 1665, the method 1600 includes determining that the fetal lie is oblique. Execution then proceeds to step 430.

In step 1660, the method 1600 includes determining that the orientation (Θ) is between - 15° and 15° or between 165° and -165°. Execution then proceeds to step 1675.

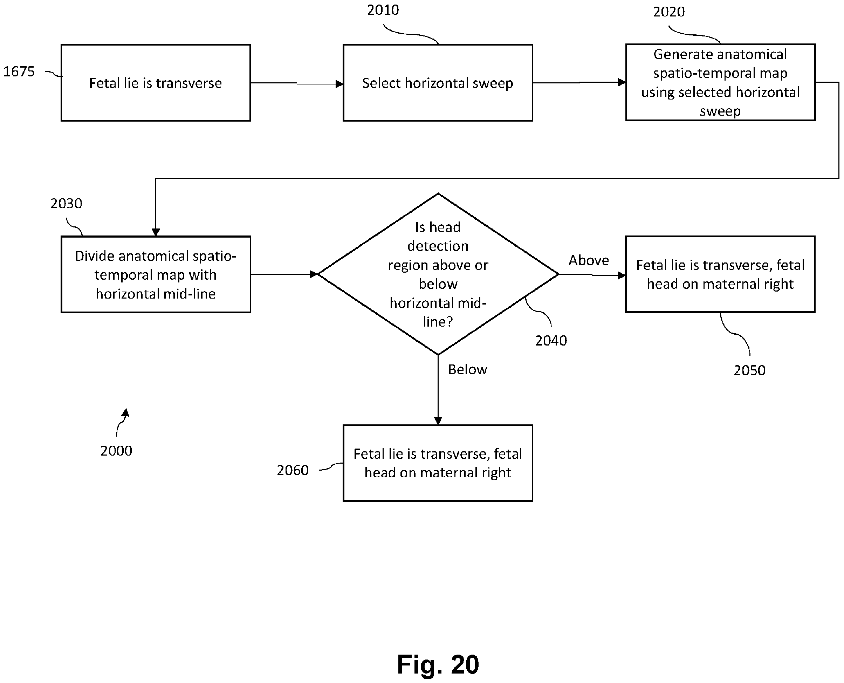

In step 1675, the method 1600 includes determining that the fetal lie is transverse. Execution then proceeds to step 2010 of Fig. 20.

In step 430, the method 1600 includes outputting a visual representation of the fetal lie identification 420. The method 1600 is now complete.

It is noted that the ranges listed for Fig. 16 are simply example values +/- 15 degrees from the ideal values. For a longitudinal lie, the ideal value is 90 degrees (breech) or -90 degrees (cephalic). For an oblique lie, the ideal values are 45 degrees, 135 degrees, -135 degrees, or -45 degrees. For a transverse lie, the ideal values are 0 degrees (head on maternal left) and 180 degrees (head on maternal right).

Fig. 16B is a schematic, diagrammatic representation, in hybrid flow diagram/block diagram form, of an example fetal lie determination method 1630, according to aspects of the present disclosure. Fig. 16B shows an expanded view of step 1630 from Fig. 16A.

In step 1631, the method 1630 begins with the angle from a line equation derived from principle component analysis as described above. Execution then proceeds to step 1632.

In step 1632, the method 1630 includes determining whether the head is above or below the abdomen and/or heart. If above, execution then proceeds to step 1633. If below, execution proceeds to step 1635.

In step 1633, the method 1630 includes determining whether the sign of the angle is less than zero. If yes, execution proceeds to step 1634. If no, execution proceeds to step 1637. In step 1634, the method 1630 includes setting the angle equal to 180 degrees minus the angle. The method 1630 is now complete.

In step 1635, the method 1630 includes determining whether the sign of the angle is greater than zero. If yes, execution proceeds to step 1636. If no, execution proceeds to step 1637. In step 1636, the method 1630 includes setting the angle to minus (180-angle). The method 1630 is now complete.

In step 1637, the method 1630 includes leaving the angle unchanged. The method 1630 is now complete.

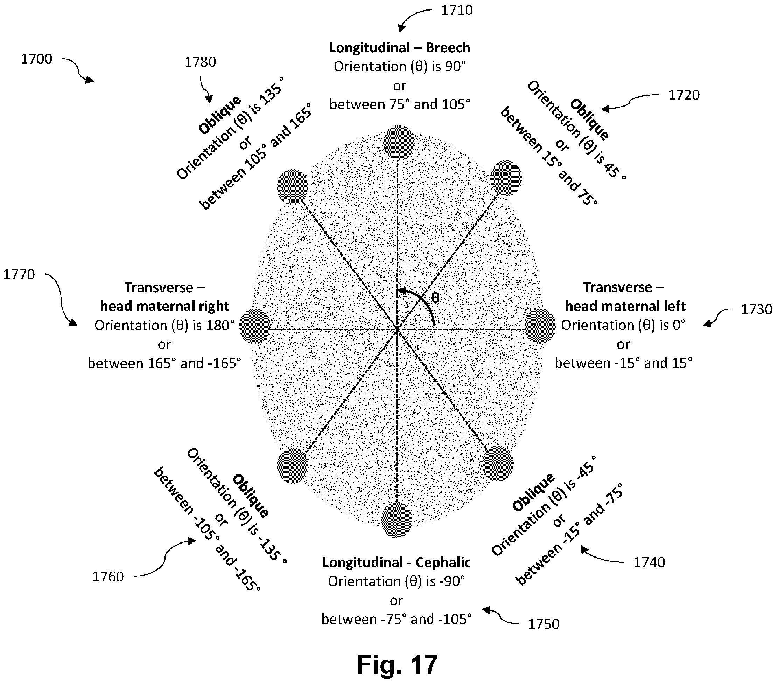

Fig. 17 is a diagram indicating the meaning of different values for the fetal orientation angle θ, according to aspects of the present disclosure. When the orientation (θ) is 90°, or between 75° and 105°, the lie is longitudinal - breech. When the orientation (Θ) is 45°, or between 15° and 75°, the lie is oblique. When the orientation (θ) is 0°, or between -15° and 15°, the lie is transverse - head maternal left. When the orientation (Θ) is -45°, or between -15° and -75°, the lie is oblique. When the orientation (θ) is -90°, or between -75° and -105°, the lie is longitudinal - cephalic. When the orientation (Θ) is - 135°, or between -105° and -165°, the lie is oblique. When the orientation (θ) is 180°, or between 165° and -165°, the lie is transverse - head maternal right. When the orientation (Θ) is 135°, or between 105° and 165°, the lie is oblique.

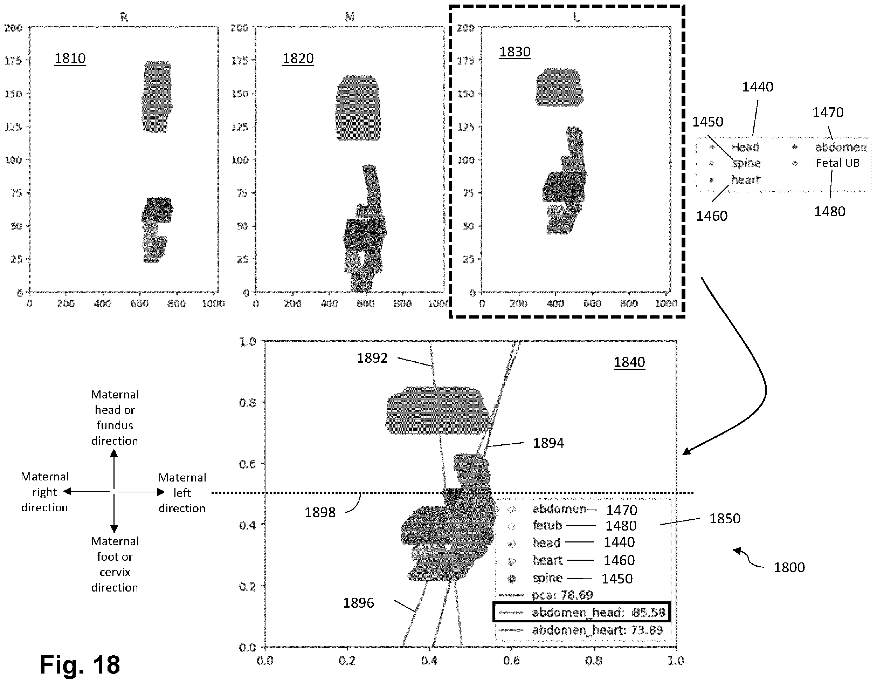

Fig. 18 is a schematic, diagrammatic representation, of an example fetal orientation determination process 1800, according to aspects of the present disclosure. Three vertical sweeps 1810, 1820, and 1830 are evaluated, and the L sweep 1830 is selected. This selection may be a count-based determination, based on the quantity of anatomy types detected and/or the total quantity of detections. As described in Fig. 11; in addition to a count-based determination, the sweep selection can also be based on where along the length of the sweep the anatomy detections begin and end. For example, in the L sweep 1830, detections are spaced from both the beginning (frame 0, which is closer to cervix) and ending (frame 200, which is closer to fundus) of the sweep; this can be favored to make sure anatomy detections are not missed/cut off. In contrast, in the M sweep, detections not spaced from the beginning of sweep; that is, detections start at frame 0, which is closer to the cervix. This may be disfavored, because there may be anatomy detections that are cut off or missed (e.g., closer towards the cervix, below the beginning of the M sweep).

The selected sweep 1840 is the L sweep 1830, and contains the same data as the L sweep 183, although it looks slightly differently because the x and y scale have been modified, and a different color-coding scheme has been used. Also, some of the anatomy regions in the L sweep at the bottom have been moved forward/backward (e.g., into or out of the page) relative to those in the L sweep at the top.

The abdomen detection region 1470 and head detection region 1440 are identified, and a line 1892 (marked "abdomen_head" in the legend 1850) is drawn between them. In this example, orientation (Θ) is the angle of that abdomen_head line, which is used to determine fetal lie. However, other lines also appear in the image. As part of the PCA process, the spine detection region 1450 is identified as the longest component, and a line 1894 (marked "pea" in the legend) is the orientation of the spine-abdomen region, such that the line 1894 passes through the spine 1450 and the abdomen 1470. A heart detection region 1460 is also identified, and a line 1896 (labeled "abdomen_heart" in the legend) is drawn between the abdomen detection region 1470 and the heart detection region. In various aspects, the abdomen-head line, the PCA line, or the abdomen-heart line could be used at the orientation (theta) used to determine fetal lie, depending which gives more accurate results, e.g., for edge cases where the fetus is in a curved orientation.

In the example shown in Fig. 18, the orientation (Θ) is calculated to be -85.58 degrees, based on the abdomen-head line, indicating a longitudinal-breech lie for the fetus. The absolute value of the orientation θ (e.g., close to 90 degrees) indicates a longitudinal lie. The sign (positive or negative) can indicate whether the longitudinal lie is cephalic or breech. This sign convention is similar to adding a horizontal mid-line and determining whether the head detection region is above the horizontal mid-line (breech) or below the horizontal mid-line (cephalic)

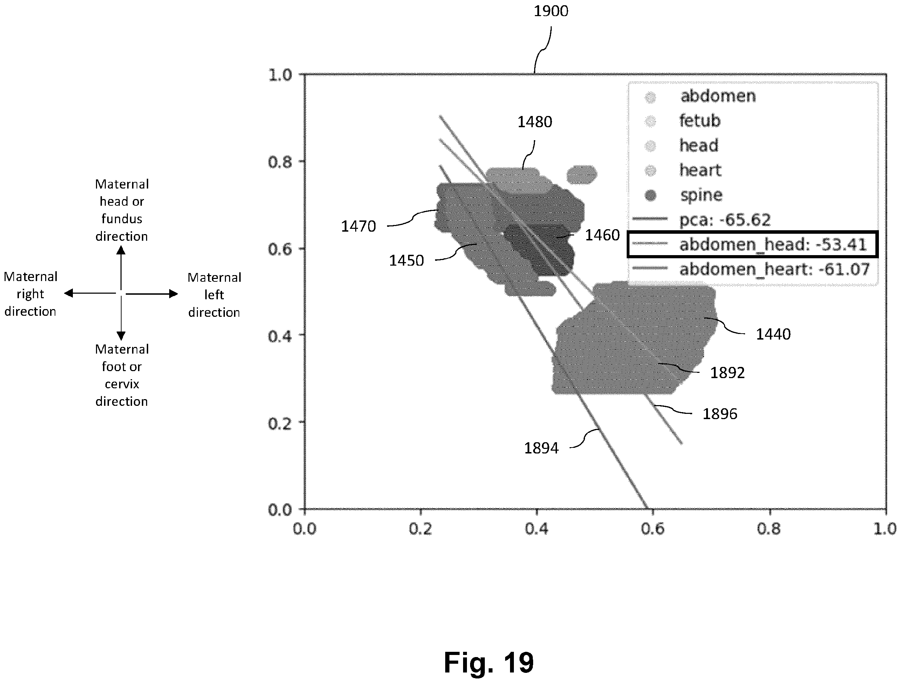

Fig. 19 is a 2D anatomical spatio-temporal map 1900 for a vertical sweep (e.g., an M sweep), according to aspects of the present disclosure. Visible are the fetal head 1440, spine 1450, heart 1460, abdomen 1470, and fetal urinary bladder 1480. Between the abdomen detection region 1470 and head detection region 1440 a line 1892 is drawn. In the example shown in Fig. 19, orientation (Θ) is the angle of that abdomen_head line 1892, which is used to determine fetal lie.

Other lines can be seen in the map 1900. As part of the PCA process, the spine detection region 1450 is identified as the longest component, and a line 1894 is drawn along the spine 1450 and passing through the abdomen 1470, thus identifying the orientation of the spine abdomen region. A heart detection region 1460 is also identified, and a line 1896 is drawn between the abdomen detection region 1470 and the heart detection region 1460. In various aspects, the PCA line 1894 or the abdomen-heart line 1896 could be used as the orientation (Θ) used to determine fetal lie, instead of the abdomen-head line 1892.

In the example shown in Fig. 19, the orientation (Θ) is -53.41 degrees, which indicates an oblique lie with the head between the transverse head maternal right and longitudinal cephalic positions (see Fig. 17).

Fig. 20 is a schematic, diagrammatic representation, in flow diagram form, of an example fetal lie detection method 2000, according to aspects of the present disclosure.

In step 1675 of Fig. 16, the method 2000 includes determining that the fetal lie is transverse. Execution then proceeds to step 2010.

In step 2010, the method 2000 includes selecting a horizontal sweep. The selection of which horizontal sweep to use could be made using a count-based technique and/or where anatomy detections begin/end (as described above in Figs. 13 and 18). Execution then proceeds to step 2020.

In step 2020, the method 2000 includes generating an anatomical spatio-temporal map using the selected horizontal sweep, using the methods described above in Fig. 14. Execution then proceeds to step 2030.

In step 2030, the method 2000 includes dividing the anatomical spatio-temporal map with a horizontal mid-line. Execution then proceeds to step 2040.

In step 2040, the method 2000 includes determining whether the head detection region (or a midpoint thereof) is above or below the horizontal mid-line. If above, execution proceeds to step 2050. If below, execution proceeds to step 2060.

In step 2050, the method 2000 includes determining that the fetal lie is transverse, with the head on the maternal right. Execution then proceeds to step 420 of Fig. 16.

In step 2060, the method 2000 includes determining that the fetal lie is transverse, with the head on the maternal left. Execution then proceeds to step 420 of Fig. 16.

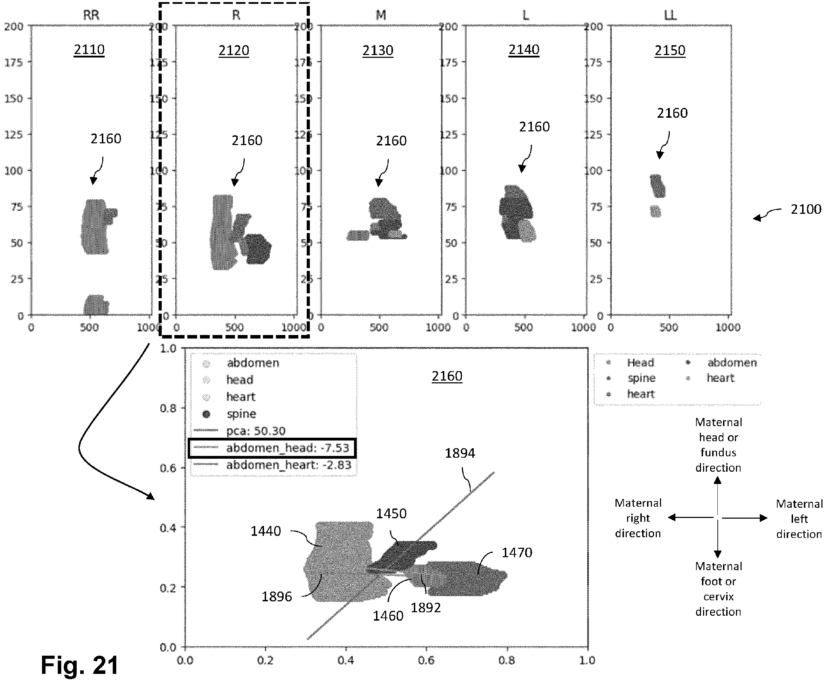

Fig. 21 is a schematic, diagrammatic representation, of an example fetal orientation determination process 2100, according to aspects of the present disclosure. Five 2D spatio-temporal maps of vertical sweeps 2110, 2120, 2130, 2140, and 2150 are evaluated, and the R sweep map 2120 is selected, based on the number of anatomy detections 2160 in the map 2120, where the anatomies begin or end, etc.

The selected map 2160 thus contains the same data as the R sweep map 2120, although it looks slightly different due to changes in the X-Y scaling, color mapping, and front-back stacking of the colors Visible in the selected sweep 2160 are the fetal head 1440, spine 1450, heart 1460, and abdomen 1470.

The abdomen detection region 1470 and head detection region 1440 are identified, and a line 1892 is drawn between them. In this example, the fetal orientation (Θ) is the angle of that abdomen-head line 1892, which is used to determine fetal lie. However, other lines appear in the image. As part of PCA process, the spine detection region 1450 is identified as the longest component, and a line 1894 (labeled "pca") is drawn along the centerline of the spine 1450. The heart detection region 1460 is also identified, and a line 1896 is drawn between the abdomen detection region 1470 and the heart detection region 1460, that is labeled "abdomen-heart". In various aspects, the PCA line 1894 or the abdomen-heart line 1896 could be used at the orientation (θ) to determine fetal lie. In the example shown in Fig. 21, the value of the orientation (theta) is -7.53 degrees. The value of the orientation (e.g., between - 15 degrees and 15 degrees) thus indicates a transverse lie.

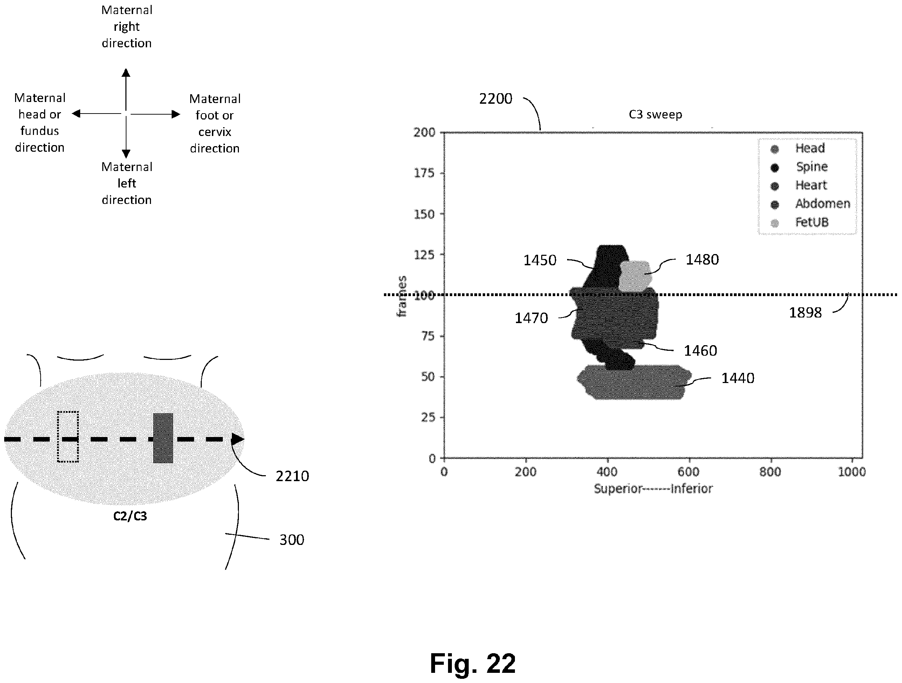

Vertical sweep data does not provide enough information to confirm whether the baby's head is to the maternal left or right with the transverse lie. Therefore, the system needs to consider a horizontal sweep, as shown below in Fig. 22. The sign (positive or negative) of θ can indicate whether the longitudinal lie is cephalic or breech. In some aspects, the line is drawn start from the abdomen detection region and going to the head detection region. When the line is going up from the abdomen detection region to get to the head detection region, the sign can be negative. This indicates that the fetal head is in the direction of the maternal head/fundus, which is a breech lie. When the line is going up from the abdomen detection region to get to the head detection region, the sign can be positive. This indicates that the fetal head is in the direction of the maternal foot/cervix, which is a cephalic lie. This sign convention is similar to adding a horizontal mid-line and determining whether the head detection region is above the horizontal mid-line (breech) or below the horizontal mid-line (cephalic).

Fig. 22 is a schematic, diagrammatic representation of an example fetal orientation determination process, according to aspects of the present disclosure. A horizontal sweep spatio-temporal map 2200 is selected based on the number and location of the detections it contains, as described in detail above. In the example shown in Fig. 22, the C2 or C3 sweep 2210 is selected. Visible are the fetal head 1440, spine 1450, heart 1460, abdomen 1470, and urinary bladder 1480.

Because the anatomical spatio-temporal map from vertical sweep indicated that fie lie is transverse (see Fig. 21, above), it is necessary to select a horizontal sweep. This can be done using a count-based determination, based on where along the length of the sweep the anatomy detections begin and end, etc. - similar to selection described in Figs. 11 and 18. All horizontal sweeps can be considered, or the consideration can be hierarchical/preferential. For example, the selection process can prefer the middle horizontal sweep (C2 when using 3x3 blind sweep protocol, or C3 when using a 5x5 blind sweep protocol). Based on experimental data, it is expected that the middle horizontal sweep (e.g., the C2 or C3 sweep) will be the selected sweep, because experimental data shows that the middle horizontal sweep usually has the most anatomy types and/or detections. However, the selection can be any horizontal sweep.

As shown in Fig. 4, a horizontal sweep in the blind sweep protocol can go from the maternal right to the maternal left. The system then generates an anatomical spatio-temporal map for the selected horizontal sweep (e.g., as shown above in Fig. 12). Next, the system estimates the position of the fetal head 1440 relative to a horizontal mid-line 1898 for the horizontal sweep. If the head detection region is below the horizontal mid-line 1898, indicates that fetal head is on maternal left (as shown in Fig. 22). Alternatively, if the head detection region 1440 is above the horizontal mid-line 1898, this indicates that the fetal head is on the maternal right.

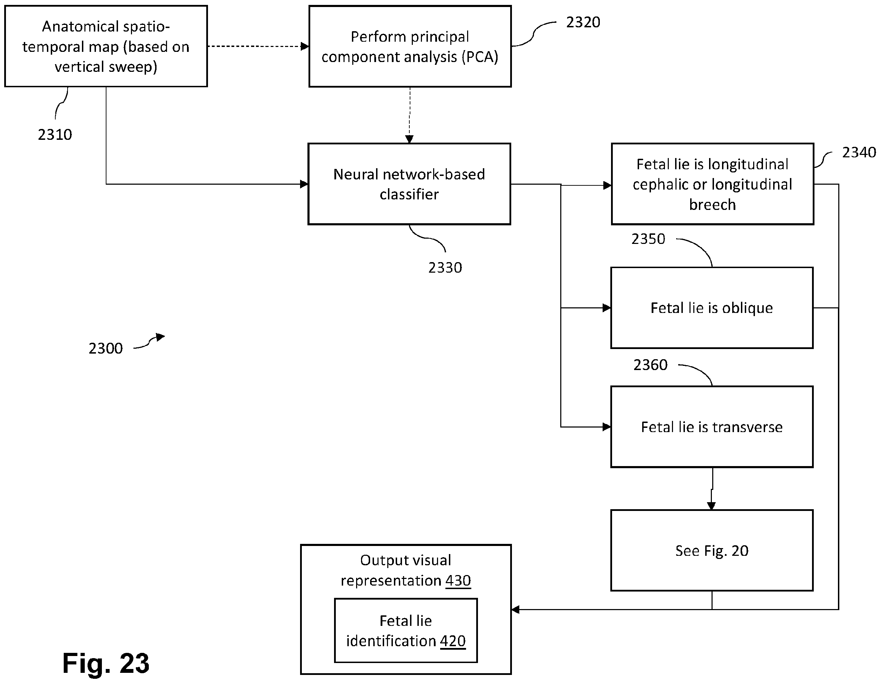

Fig. 23 is a schematic, diagrammatic representation, in hybrid flow diagram/block diagram form, of an example fetal lie determination method 2300, according to aspects of the present disclosure. A predictive model 2330 (e.g., a neural network-based classifier such as a convolutional neural network (CNN) or state vector machine (SVM)) receives, as an input, either the selected anatomical spatio-temporal map 2310 itself (e.g., selected from the available vertical sweeps), or else the components of a principal component analysis (PCA) 2320 performed on the map 2310, or a combination thereof. The classifier outputs a fetal lie prediction 2340, 2350, or 2360. It is noted that for classifying cephalic, breech, etc., a heuristic may be used instead or in addition,

In step 2340, the classifier 2330 has determined that the fetal lie is longitudinal cephalic or longitudinal breech. Execution then proceeds to step 430.

In step 2350, the classifier 2330 has determined that the fetal lie is oblique. Execution then proceeds to step 430.

In step 2360, the classifier 2330 has determined that the fetal lie is transverse. Execution then proceeds to step 2010 of Fig. 20.

In step 430, the method 2300 includes outputting a visual representation of the fetal lie identification 420. The method 2300 is now complete.

Examples of the classifier 2330 include CNN, SVM, etc. In some aspects, an ensemble approach may be used to determine the fetal lie, using a combination of predictive model (as shown here in Fig. 23) and the PCA (as shown in Fig. 16), such as the head-abdomen line (as shown in Fig. 18).

Fig. 24 is a schematic, diagrammatic representation of an example fetal orientation determination method 2400, according to aspects of the present disclosure. The method 2400 assumes that fetal lie has already been determined in Figs. 16-23.

The purpose of the method 2400 is to determine whether the fetal spine is anterior or posterior. This requires the sweep direction (vertical or horizontal) that will provide ultrasound data necessary to determine fetal spine presentation. This will depend on the fetal lie, e.g., the sweep direction used to determine fetal spine presentation should be perpendicular to the fetal lie. That is, to determine fetal spine presentation, the sweep direction crosses perpendicular across the fetus's body (not along the length of fetus's body-this is what is used to determine fetal lie, as described in Figs. 16, 23, etc.) The sweep direction crossing perpendicular across the fetus's body provides information about the fetal spine in relation to other anatomy in the uterus, which includes both other fetal anatomy (e.g., fetal heart) and maternal anatomy (e.g., placenta, amniotic fluid).

In contrast, the sweep direction extending along the length of the fetus's body is used to determine fetal lie in Figs. 16-23 (e.g., a vertical sweep parallel to/coaxial with and used to determine a longitudinal lie, a horizontal sweep is parallel to/coaxial with and used to determine a transverse lie). This provides information about the spatial relationship of the fetal anatomies to one another, which is what fetal lie (how is the fetal anatomies arranged).

In step 2410, the fetal lie is longitudinal, and a horizontal sweep is selected. Execution then proceeds to step 2420.

In step 2420, the method 2400 includes receiving the bounding boxes or masks for the anatomy detections in the selected horizontal sweep. Execution then proceeds to step 2430.

In step 2440, the fetal lie is transverse, and a vertical sweep is selected. Execution then proceeds to step 2450.

In step 2450, the method 2400 includes receiving the bounding boxes or masks for the anatomy detections in the vertical sweep. Execution then proceeds to step 2430.

In step 2430, the method 2400 includes determining the midpoints of the bounding boxes for each anatomy type (e.g., head, spine, heart, abdomen, placenta, amniotic fluid, etc.). Execution then proceeds to step 2460.

In step 2460, the method includes generating a plot of the midpoints. The plot thus includes clusters of midpoints of each respective anatomy type. Execution then proceeds to steps 2470 and 2480.

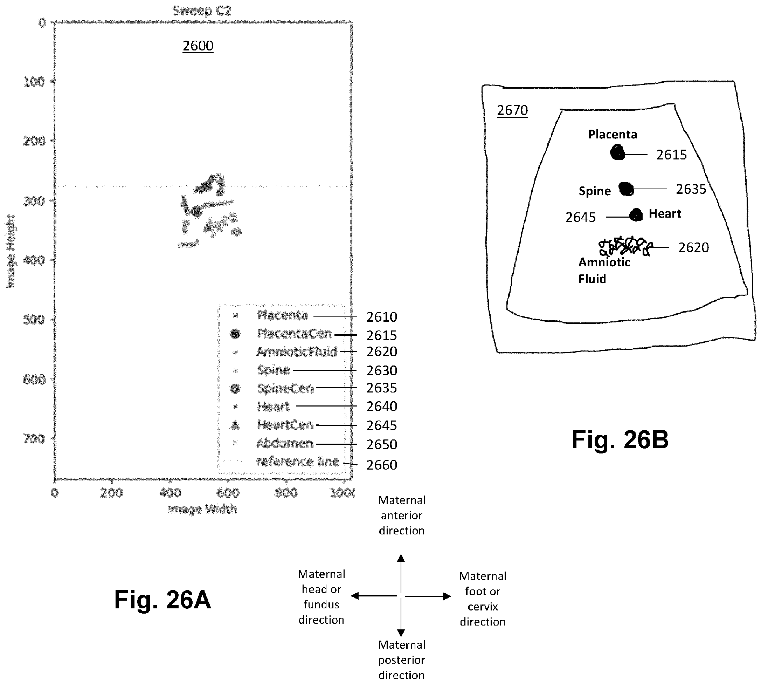

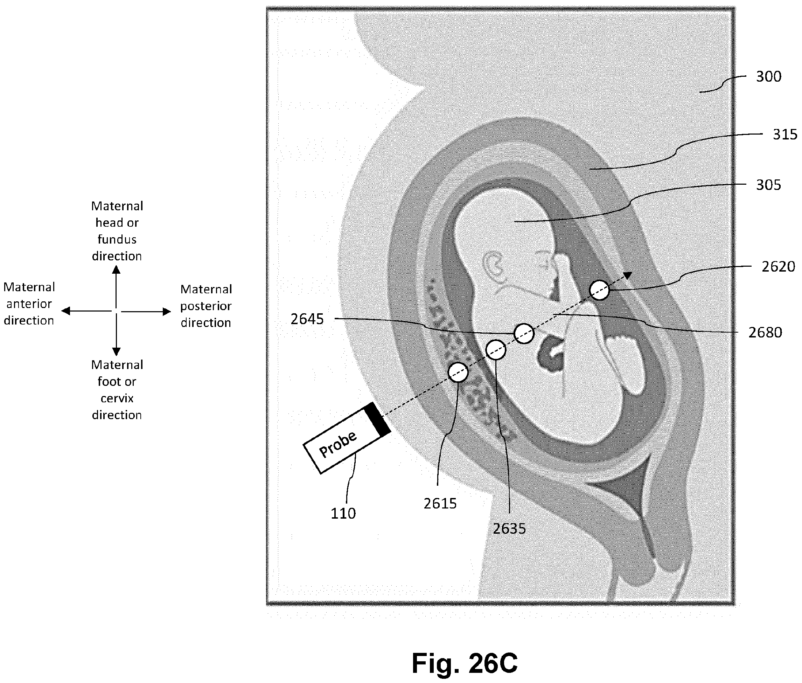

The plot of mid-points may be representative of the sweep as a whole. For example, an individual ultrasound frame can have no detections, can have detections of one anatomy type, or multiple detections of multiple anatomy types. The plot includes the mid-point of each of these bounding boxes, repeated for all ultrasound image frames in the sweep. All of the mid-points are thus plotted. In the plot, the mid-points can be grouped based on anatomy type. The mid-points of respective anatomy types can form clusters. The centroid is the center of grouping/cluster of mid-points for a respective anatomy type. See the example plots in Figs. 26 and 28 for details.

In step 2470, the method 2400 includes determining the centroids of the fetal spine cluster, the fetal heart cluster, and the placenta cluster. Execution then proceeds to step 2490.

In step 2480, the method 2400 includes determining the placenta location with respect to the amniotic fluid to determine whether the placenta is in an anterior or posterior position. Execution then proceeds to step 2490.

Determining placenta location with respect to amniotic fluid is described in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein.

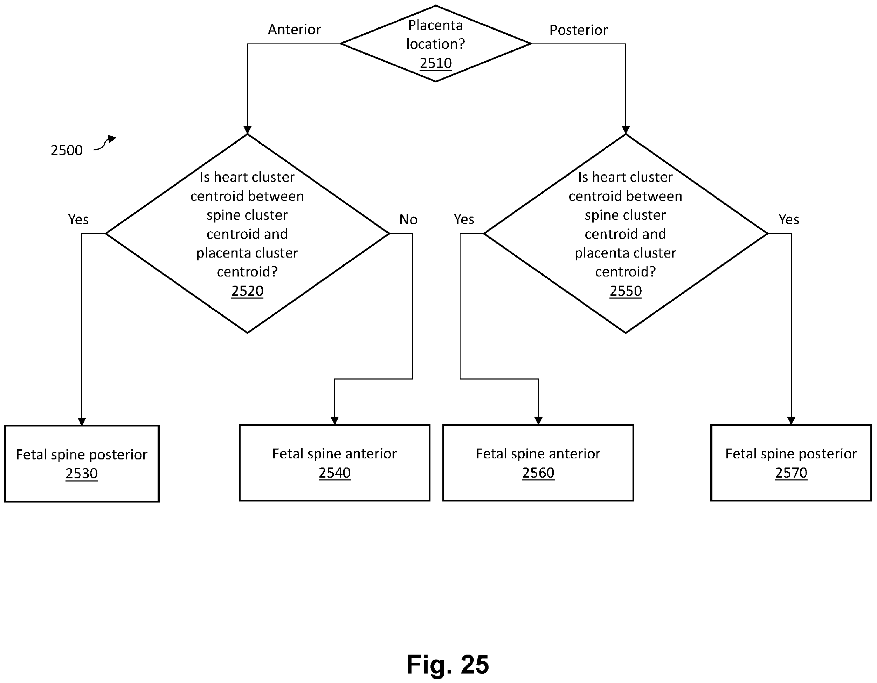

In step 2490, the method 2400 includes estimating the fetal spine position, based on the placenta location and the positions of the spine cluster centroid, heart cluster centroid, and placenta cluster centroid, as described in Fig. 25, below. Execution then proceeds to step 2495.

In step 2495, the method 2400 includes determining, based on the fetal spine position, whether the fetal presentation is fetal spine anterior or fetal spine posterior. Execution then proceeds to step 2499.

In step 2499, the method 2400 includes outputting a visual representation that includes the fetal spine 1450 and/or the fetal spine presentation 2495. Depending on the implementation, the visual representation may also include the plot of mid-points from step 2460.

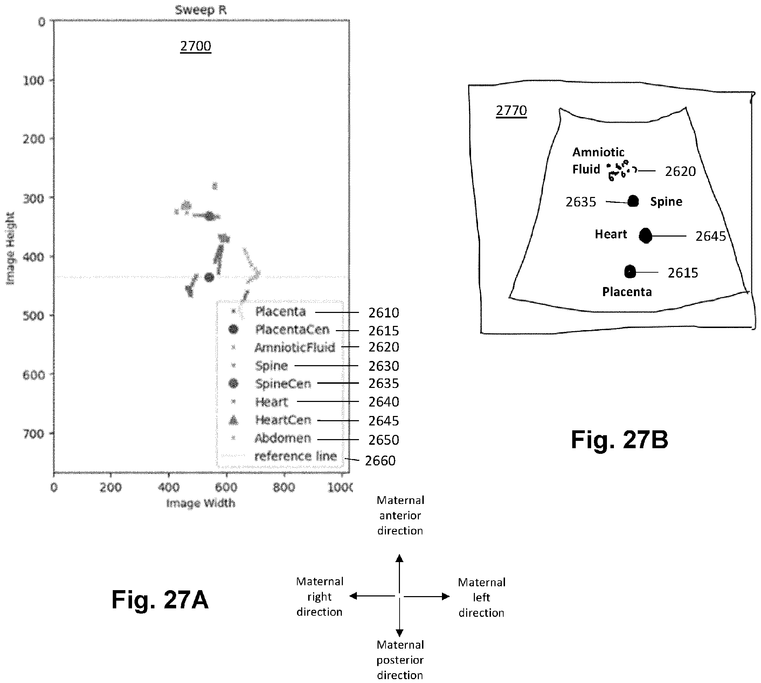

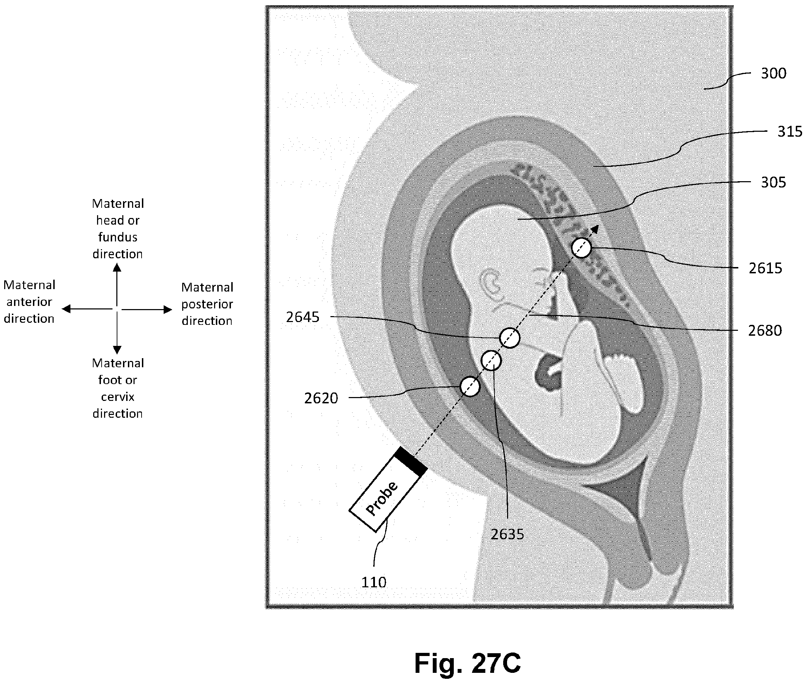

Fig. 25 is a schematic, diagrammatic representation, in flow diagram form, of an example fetal spine presentation determination method 2500, according to aspects of the present disclosure. As a whole, this flow diagram is using the expected spatial relationships (positioning) between placenta, amniotic fluid, fetal heart, and fetal spine to determine the fetal spine presentation. For example, when the placenta is anterior and the fetal spine is posterior, the expectation is that fetal heart will not be between the placenta and the fetal spine. Rather, the fetal spine will between the placenta and the fetal heart. (See Figs. 26 and 27.)

Similarly, when the placenta is posterior and the fetal spine is anterior, the expectation is that the expectation is that the fetal heart will be between the fetal spine and the placenta. (See Figs. 28 and 29.) When the placenta is posterior and the fetal spine is posterior, the expectation is that the fetal heart will be between the placenta and the fetal spine. When the placenta is anterior and the fetal spine is anterior, the expectation is that the fetal heart will not be between the placenta and the fetal spine. Rather, the fetal spine will between the placenta and the fetal heart.

In step 2510, the method 2500 includes determining whether the placenta location is anterior or posterior. If anterior, execution proceeds to step 2520. If posterior, execution proceeds to step 2550.

In step 2520, the method 2500 includers determining whether the heart cluster centroid is between the spine cluster centroid and the placenta cluster centroid. If yes, execution proceeds to step 2530. If no, execution proceeds to step 2540.

In step 2530, the method 2500 includes determining that the fetal spine presentation is posterior. The method 2500 is now complete.

In step 2540, the method 2500 includes determining that the fetal spine presentation is anterior. The method 2500 is now complete.

In step 2550, the method 2500 includes determining whether the heart cluster centroid is between the spine cluster centroid and the placenta cluster centroid. If yes, execution proceeds to step 2560. If no, execution proceeds to step 2570.

In step 2560, the method 2500 includes determining that the fetal spine presentation is anterior. The method 2500 is now complete.

In step 2570, the method 2500 includes determining that the fetal spine presentation is posterior. The method 2500 is now complete.

Fig. 26A is a plot 2600 of the midpoints of the anatomy detection boxes across a sweep, according to aspects of the present disclosure. In the example shown in Fig. 26A, the fetus has a longitudinal lie, and therefore a horizontal sweep (e.g., the C2 sweep) is selected. Visible are the placenta midpoints 2610, centroid of the placenta midpoints 2615, the amniotic fluid midpoints 2620, the spine midpoints 2630, centroid of the spine midpoints 2635, heart midpoints 2640, centroid of the heart midpoints 2645, abdomen midpoints 2650, and a reference line 2660 (which may for example represent the average of the centroids of the amniotic fluid midpoints). The centroid of each detection cluster is a handy surrogate for the center of the anatomy itself, such that the heart centroid 2645 may be presumed to be close to the center of the heart, the placenta centroid 2615 may be presumed to be close to the center of the placenta, etc.

Determining placenta location with respect to amniotic fluid is described for example in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein.

Fig. 26B is a synthesized ultrasound image 2670 generated based on the anatomy detection cluster centroids of Fig. 26A, according to aspects of the present disclosure. As can be seen in the image, the placenta centroid 2615 is in an anterior position, and the heart centroid 2645 is not located between the spine centroid 2635 and the placenta centroid 2615. Per Fig. 25, above, this arrangement of features indicates a fetal spine anterior presentation. Depending on the implementation, the synthesized ultrasound image 2670 may be shown to the user, along with a text indication of the fetal spine presentation, as part of the output visual representation 2499 of Fig. 24.

Fig. 26C is a schematic, diagrammatic, side cross-sectional view of a fetus 305 inside a uterus 315 of a patient 300, according to aspects of the present disclosure. The dotted line 2680 represents an imaging plane for the imaginary or synthetic ultrasound image in Fig. 26B. Thus, Fig. 26C is representative of the geometry of Figs. 26A and 26B, with fetus 305 in a longitudinal, breech lie, with anterior placenta 2615 and anterior presentation of the spine 2635. Thus, an ultrasound probe 110 placed on the abdomen of the patient first sees the placenta centroid 2615 as the shallowest feature in an ultrasound image, then the spine centroid 2635, then the heart centroid 2645, with the amniotic fluid 2620 being the deepest feature in the image.

Fig. 27A is a plot 2700 of the midpoints of the anatomy detection boxes across a sweep, according to aspects of the present disclosure. In the example shown in Fig. 27A, the fetus has a transverse lie, and therefore a Vertical sweep (e.g., the M sweep) is selected. Visible are the placenta midpoints 2610, centroid of the placenta midpoints 2615, the amniotic fluid midpoints 2620, the spine midpoints 2630, centroid of the spine midpoints 2635, heart midpoints 2640, centroid of the heart midpoints 2645, abdomen midpoints 2650, and a reference line 2660.

Fig. 27B is a synthesized ultrasound image 2770 generated based on the anatomy detection cluster centroids of Fig. 27A, according to aspects of the present disclosure. As can be seen in the image, the placenta centroid 2615 is in a posterior position, and the heart centroid 2645 is located between the spine centroid 2635 and the placenta centroid 2615. Per Fig. 25, above, this arrangement of features indicates a fetal spine anterior presentation. Depending on the implementation, the synthesized ultrasound image 2670 may be shown to the user, along with a text indication of the fetal spine presentation, as part of the output visual representation 2499 of Fig. 24.

Fig. 27C is a schematic, diagrammatic, side cross-sectional view of a fetus 305 inside a uterus 315 of a patient 300, according to aspects of the present disclosure. The dotted line 2680 represents an imaging plane for the imaginary or synthetic ultrasound image in Fig. 27B. Thus, Fig. 27C is representative of the geometry of Figs. 27A and 27B, with the fetus 305 in a longitudinal, breech lie, with posterior placenta 2615 and anterior presentation of the spine 2635. Thus, an ultrasound probe 110 placed on the abdomen of the patient 300 first sees the amniotic fluid 2620 as the shallowest feature in an ultrasound image, then the spine centroid 2635, then the heart centroid 2645, with the placenta centroid 2615 being the deepest feature in the image.

Fig. 28 is a schematic, diagrammatic representation of a sweep acceptance process 2800, according to aspects of the present disclosure. In the example shown in Fig. 28, a horizontal calibration sweep 2810 and a vertical calibration sweep 2820 each cover the entire width and height of the abdomen 310, respectively, thus providing full coverage of the fetus 305. Thus, the calibration sweeps 2810, 2820 are accepted, and the user is permitted to begin the blind sweep protocol.

Fig. 29 is a schematic, diagrammatic representation of a sweep rejection process 2900, according to aspects of the present disclosure. In the example shown in Fig. 28, a horizontal calibration sweep 2910 and a vertical calibration sweep 2920 each cover only a portion of the entire width and height of the abdomen 310, respectively, thus providing only partial coverage of the fetus 305. Thus, the calibration sweeps 2910, 2920 are rejected, and the user is instructed to repeat the calibration sweeps 2910, 2920 before the blind sweep protocol can begin.

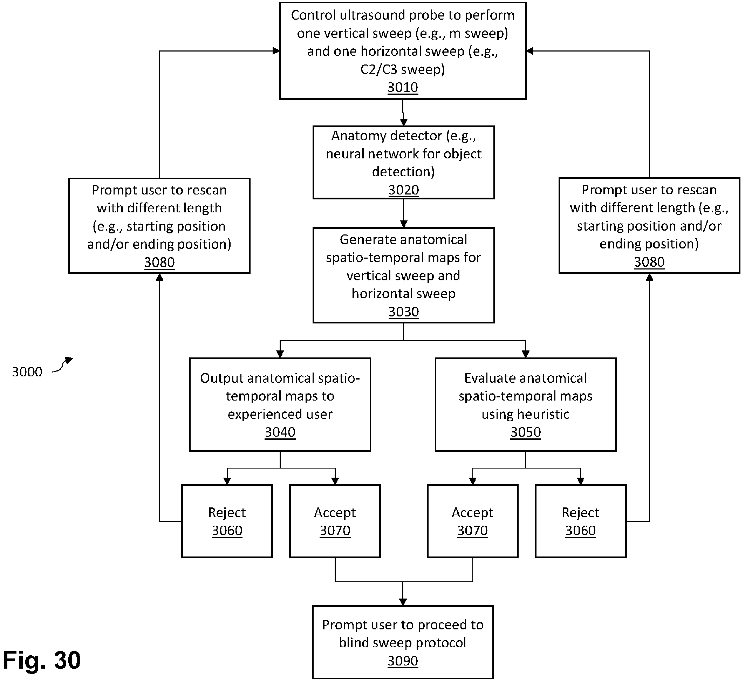

Fig. 30 is a schematic, diagrammatic representation, in hybrid flow diagram / block diagram form, of an example sweep calibration method 3000, according to aspects of the present disclosure. The purpose of the calibration method 3000 is to ensure the user understands how to perform a proper vertical sweep and a proper horizontal sweep, such that the entire fetus is imaged by at least one of the sweeps.

In step 3010, the method 3000 includes controlling the ultrasound probe to perform one vertical sweep (e.g., the M sweep) and one horizontal sweep (e.g., the C2 sweep in the case of a 3x3 protocol, or the C3 sweep in the case of a 5x5 protocol). Execution then proceeds to step 3020.

In step 3020, the method 3000 includes passing the sweeps to the anatomy detector to generate bounding boxes around detected fetal and/or maternal anatomy. Execution then proceeds to step 3030.

In step 3030, the method 3000 includes generating anatomical spatio-temporal maps for the vertical sweep and the horizontal sweep (e.g., as described above in Figs. 12A - 14). Execution then proceeds to step 3040 or step 3050, depending on whether an experienced user is available.

In step 3040, the method 3000 includes outputting the anatomical spatio-temporal maps to the experienced user, who can then manipulate a user control to accept or reject the sweeps. If reject, execution proceeds to step 3060. If accept, execution proceeds to step 3070.

In step 3050, the method 3000 includes evaluating the spatio-temporal maps using heuristics to determine whether full coverage of the fetus has been achieved. If yes, execution proceeds to step 3070. If no, execution proceeds to step 3060. In a non-limiting example, a vertical sweep may be rejected if the cervix is not detected. This is because cervix detections are expected at the beginning of vertical sweeps that have a correct starting location (low enough on the maternal abdomen/belly. In another non-limiting example, the sweeps may be accepted only if there are anatomy type detections from the fetal head to the fetal urinary bladder (e.g., a majority or full length of the fetus). In the calibration step, scan acceptance and rejection can be an automated or manual process. In case of manual, the system can directly show the visual map to the user. If the scan captures sufficient anatomies, it will be accepted; otherwise it will be rejected. In case of automation, a heuristic approach can be used where the system can check whether the cervix (and all other fetal anatomies like head, heart, abdomen, fetus urinary bladder) are captured. If so, accept the scan, and otherwise reject it. Automation may be preferred in the case of an inexperienced user such as a midwife.

In step 3060, the calibration sweeps are rejected. Execution then proceeds to step 3080.

In step 3070, the calibration sweeps are accepted. Execution then proceeds to step 3090. In step 3080, the method 3000 includes prompting the user to rescan the patient's abdomen using different lengths (e.g., using different starting positions and/or different ending positions). Execution then returns to step 3010.

In step 3090, the method 3000 includes prompting the user to begin the blind sweep protocol. The method 3000 is now complete.

Fig. 31 is a schematic, diagrammatic representation of accepted or acceptable calibration sweeps, according to aspects of the present disclosure. A vertical sweep 3110 (e.g., an M sweep) and a horizontal sweep 3120 (e.g., a C2 or C3 sweep) each contain significant detections of the fetal head 1440, spine 1450, heart 1460, abdomen 1470, and urinary bladder 1480. Thus, it can be deduced that the calibration sweeps were performed correctly, and that the user can proceed to the blind sweep protocol.

Fig. 32 is a schematic, diagrammatic representation of rejected or unacceptable calibration sweeps, according to aspects of the present disclosure. A vertical sweep 3210 contains significant detections of the fetal head 1440 and spine 1450, but not of the heart, abdomen, or urinary bladder. Another vertical sweep 3220 (e.g., an L sweep) contains significant detections of the abdomen 1470, urinary bladder 1480, and spine 1450, but not of the heart or head. A horizontal sweep 3230 (e.g., a C2 or C3 sweep) contains significant detections of the head 1440, heart 1460, and abdomen 1470, but contains no detections of the fetal urinary bladder, and contains multiple, physically separated detections of the fetal spine 1450. Thus, it can be deduced that the calibration sweeps were performed incorrectly, and that the user must repeat the calibration sweeps before proceeding to the blind sweep protocol.

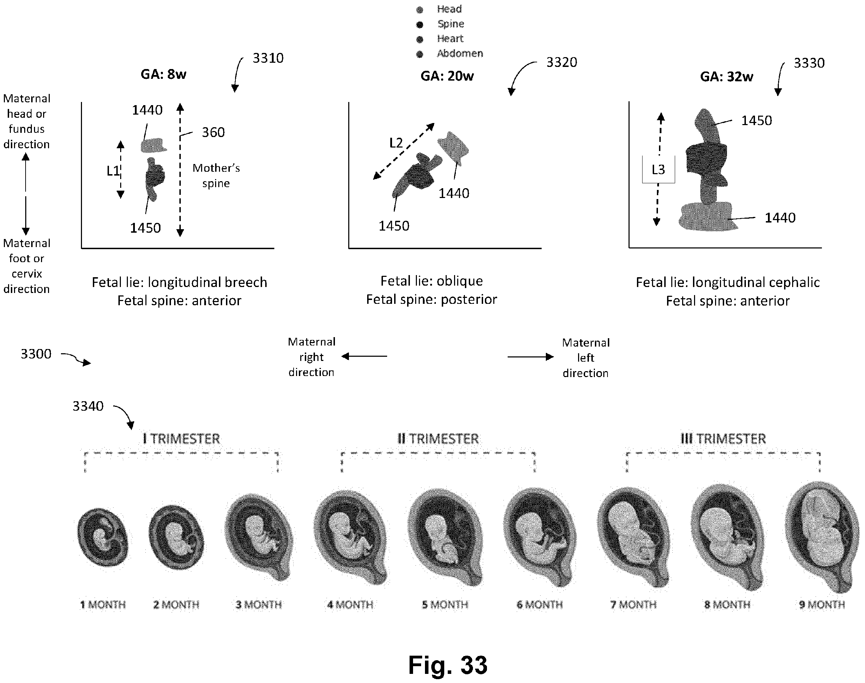

Fig. 33 is a schematic, diagrammatic representation of a fetal growth monitoring process 3300, according to aspects of the present disclosure. A spatio-temporal map 3310 of a scan performed at a gestational age of 8 weeks (2 months) shows a fetal length L1, driven by the distance between opposite ends of the head detection 1440 and spine detection 1450. Similarly, a spatio-temporal map 3320 of a scan performed at a gestational age of 20 weeks (5 months) shows a fetal length L2, which is larger than L1, and a spatio-temporal map 3330 of a scan performed at a gestational age of 32 weeks (8 months) shows a fetal length L3, which is larger than L1 or L2. These lengths can for example be measured directly by the system and displayed to the user, or can be estimated by the user based on an output to a display of the spatio-temporal maps 3310, 3320, 3330, etc., wherein the detected anatomies are growing larger as the gestational age advances. In either case, the length can be compared against a fetal size chart 3340 to determine whether the fetal length is within expected parameters for the given trimester and gestational age.

As will be readily appreciated by those having ordinary skill in the art after becoming familiar with the teachings herein, the fetal orientation detection system advantageously permits untrained and minimally trained users to perform an ultrasound blind sweep protocol to gather reliable information about the lie and presentation of the fetus. This may result in higher accuracy and higher clinician trust in the results, while potentially improving health outcomes and/or decreasing the total cost of care.

The systems, methods, and devices described herein may be applicable in point of care and handheld ultrasound use cases such as with the Philips Lumify system. The fetal orientation detection system can be used for any handheld imaging applications, including but not limited to obstetrics, prenatal lung imaging, and prenatal echocardiography. The fetal orientation detection system could be deployed on handheld mobile ultrasound devices, and on portable or cart-based ultrasound systems. The fetal orientation detection system can be used in a variety of settings, including emergency departments, ambulances, accident sites, and homes. The applications could also be expanded to other settings. Thus, the present disclosure increases the value proposition of ultrasound applications in the obstetrics context, especially for use by minimally trained users.

A number of variations are possible on the examples and aspects described above. For example, the systems, methods, and devices described herein are not limited to obstetric ultrasound applications. Rather, the same technology can be applied to images of other organs or anatomical systems such as the lungs, heart, brain, digestive system, vascular system, tumors, etc. Furthermore, the technology disclosed herein is also applicable to other medical imaging modalities where 3D data are available, such as other ultrasound applications, camera-based videos, X-ray videos, and 3D volume images, such as computer aided tomography (CT) scans, magnetic resonance imaging (MRI) scans, or optical coherence tomography (OCT) scans.

Accordingly, the logical operations making up the aspects of the technology described herein are referred to variously as operations, steps, objects, layers, elements, components, algorithms, or modules. Furthermore, it should be understood that these may occur or be performed or arranged in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the fetal orientation detection system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the fetal orientation detection system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. A system, comprising:
a processor configured for communication with an ultrasound probe, wherein the processor is configured to:
control the ultrasound probe to obtain a plurality of ultrasound image frames during a blind sweep protocol on a pregnant patient;
provide the plurality of ultrasound image frames as an input to a deep learning network trained to detect a plurality of fetal anatomies;
generate, as an output of the deep learning network, a plurality of detections of the plurality of fetal anatomies of a fetus within the plurality of ultrasound image frames;
determine a fetal lie using the detection of the plurality of fetal anatomies; and
provide, to a display in communication with the processor, an output representative of the fetal lie.

2. The system of claim 1, wherein the fetal lie comprises one of a longitudinal lie, a transverse lie, or an oblique lie.

3. The system of claim 2, wherein the longitudinal lie comprises cephalic or breech.

4. The system of claim 2, wherein the transverse lie comprises:
fetal head on maternal right; or
fetal head on maternal left.

5. The system of any of claims 1-4,
wherein the blind sweep protocol comprises a vertical sweep,
wherein the processor is configured to:
generate a plurality of first binary masks based on the plurality of detections in the vertical sweep, wherein an individual first binary mask is associated with an individual ultrasound image frame in the vertical sweep; and
generate a first map based on the plurality of first binary masks, wherein the first map is representative of the vertical sweep as a whole,
wherein the processor is configured to determine the fetal lie using the first map.

6. The system of claim 5, wherein the output representative of the fetal lie comprises the first map.

7. The system of claim 5 or 6,
wherein the processor is configured to:
generate a report comprising the first map and a previous first map; and
output to the report to the display,
wherein the first map and the previous first map each comprise a region representative of a fetal anatomy,
wherein a size of the region in the first map relative to a size of the region in the previous first map is configured to provide indication of a growth of the fetus.

8. The system of any of claims 5-7,
wherein the first map comprises a first region representative of a first fetal anatomy and a second region representative of a different, second fetal anatomy,
wherein, to determine the fetal lie using the first map, the processor is configured:
perform principle component analysis, PCA, on the first map;
generate, based on the PCA, a line between the first region and the second region; and
determine an orientation of the line; and
determine the fetal lie based on the orientation of the line.

9. The system of any of claims 5-8,
wherein the blind sweep protocol comprises a horizontal sweep,
wherein, when the processor determines the fetal lie is a transverse lie, the processor is configured to determine, based on the horizontal sweep, if the transverse lie is:
fetal head on maternal right; or
fetal head on maternal left.

10. The system of claim 9,
wherein the map comprises a first region representative of a fetal head,
wherein, to determine if the transverse lie is fetal head on maternal right or fetal head on maternal left, the processor is configured to:
generate a plurality of second binary masks based on the plurality of detections in the horizontal sweep;
generate a second map based on the plurality of second binary masks, wherein the second map is representative of the horizontal sweep; and
generate a horizontal mid-line in the second map;
determine that the transverse lie is:
fetal head on maternal right when the first region is below the horizontal mid-line; and
fetal head on maternal left when the first region is above the horizontal mid-line.

11. The system of any of the preceding claims,
wherein the deep learning network is additionally trained to detect a plurality of maternal anatomies,
wherein the processor is further configured to:
generate, also as the output of the deep learning network, a plurality of detections of the plurality of maternal anatomies within the plurality of ultrasound image frames; and
determine a fetal spine position using the plurality of detections of the plurality of fetal anatomies and the plurality of detections of the plurality of maternal anatomies, and
wherein the output is additionally representative of the fetal spine position.

12. The system of any of the preceding claims, wherein the processor is configured to:
control the ultrasound probe to perform a calibration sweep before the blind sweep protocol;
determine if a length of the calibration sweep is acceptable;
prompt a user to rescan when the length of the calibration sweep is not acceptable; and
prompt the user to proceed to the blind sweep protocol when the length of the calibration sweep is acceptable.

13. A method for characterizing a fetal position using ultrasound data from a blind abdominal imaging sweep, comprising:
controlling an ultrasound probe to obtain a plurality of ultrasound image frames during a blind sweep protocol on a pregnant patient;
providing the plurality of ultrasound image frames as an input to a deep learning network trained to detect a plurality of fetal anatomies;
generating, as an output of the deep learning network, a plurality of detections of the plurality of fetal anatomies of a fetus within the plurality of ultrasound image frames;
determining a fetal lie using the detection of the plurality of fetal anatomies; and
providing, to a display, an output representative of the fetal lie.

14. A non-transitory computer-readable medium storing instructions that, when executed by a processor, cause the processor to perform the method of claim 13.
